# EUROPEAN PATENT APPLICATION

(11) **EP 4 751 737 A2**
(43) Date of publication of application: **03.06.2026**
(21) Application number: 26172713.5
(22) Date of filing: 31.08.2022
(51) Int. Cl.: A61K 47/36

(54) **MUCOADHESIVE POLYMERS FOR NASAL DRUG DELIVERY**

(30) Priority: 31.08.2021 US 202163260738 P; 24.11.2021 US 202163264539 P; 23.02.2022 US 202263268384 P
(62) Divisional of application: 22768986.6
(71) Applicant: POLYRIZON LTD., 4366507 Raanana (IL)
(72) Inventor: IZRAELI, Tomer, Raanana (IL); TURGEMAN, Tidhar, Ramat-Gan (IL); RON, Eyal, Massachusetts (US)
(74) Representative: Boult Wade Tennant LLP

(57) **Abstract**

The invention generally concerns with mucosal protective agents, and specifically, to the agents acting on the nasal mucosa. To that end, the invention provides a series of mucoadhesive polymeric compositions and methods, which can be used per se as blocking agents against a wide range of microbial pathogens and allergens, or as smart drug delivery systems of actives through the oral and/or nasal mucosa.

## Description

### TECHNOLOGICAL FIELD

The invention generally pertains to the field of mucoadhesive polymers and specifically, hydrophilic biopolymers and natural hydrophilic polysaccharides with mucoadhesive affinities to various mucosal tissues.

### BACKGROUND

The emergence of innovative drug delivery systems along with implementation of non-invasive and painless administration routes has revolutionized the pharma industry and our approaches to treating diseases. Drug delivery systems that are administrable via mucosa have gained increasing interest due to the option to overcome the main limitations of oral administration, such as first-pass metabolism, p-gp efflux and others, and to provide high patient compliance. In addition, mucosal layers have ubiquitous presence on many organs and relatively high substance permeability. Thus, engineering new drug delivery systems and formulations that target mucosa has become one of the main focuses of pharma R&D, and specifically the mucoadhesive dosage forms to provide longer administration times and increased local and systemic bioavailability of actives.

Specific examples are the mucosa of throat and nose. Both serve as critical portals of entry of pathogens such as allergens, viruses and bacteria, and also sources of their dissemination. In the COVID-19 (Coronavirus Disease 2019) pandemic for example, a substantial amount of evidence suggests that the titers of SARS-CoV-2 (Severe Acute Respiratory Syndrome Coronavirus 2) in the throat and nose are extremely high ¹, and so is the expression of the receptor responsible for SARS-CoV-2 entry, the hACE2 receptor, in the oral cavity and nasal tissues mucosa ².

One characteristic of the COVID-19 pandemic is manifested in a rapid spread of SARS-CoV-in the global immunologically naive human population, primarily by airborne droplets and contact transmission. Another is that the clinical symptoms of COVID-19 exhibit a wide range of symptom severity, with high fatality rates in the elderly and immune-compromised patients and patients with certain comorbid conditions, on one hand, and a significant proportion of individuals with asymptomatic or subclinical disease, on the other. This latter has been estimated at 44% of the infections, worldwide.

Moreover, in terms of viral load in the nasal and oral cavities or nasopharynx, the symptomatic and asymptomatic individuals appear to be similar in that both contain some of the highest viral loads in these body parts. In addition, it has been shown that in the symptomatic individuals, the contagiousness via nasal and oral secretions is the highest before or shortly after the onset of symptoms. Thus, individuals with asymptomatic and early symptomatic COVID-19 are actually "silent spreaders" who unknowingly contribute to the exponential spread of the disease.

Thus, in addition to the conventional mitigation strategies for prevention of viral spread and infection such as vaccination, social distancing, testing, and travel restrictions, another approach would be to use agents that block or attenuate viral entry via nasal and oral openings, or agents capable of reducing viral load in the nasal and oral cavities. This type of strategy could be equally applicable on individual and populational levels and could be especially relevant to populations and sub-groups who are intolerable to vaccination or where vaccination is inaccessible. It becomes even more relevant in view of the continuous emergence of new SARS-CoV-2 variants (α, β, δ, Omicron and others) and the resulting uncertainties as to the effectiveness of existing vaccines.

From a broader perspective, the oral and nasal mucosa constitute structural and functional interface between the outside and the inside of a human body, serving as the first barrier against continuous flow of inhaled pathogens, allergens, and other substances. Important structural components of the epithelial barrier playing a critical role in the maintenance of its functionality are so called tight junctions (TJs), cell-cell junctional complexes located on the apical side of epithelial cells. There is an increasing body of evidence showing that dysfunctionality of oral and nasal epithelial barriers, and of TJs in particular, underlies many allergic diseases such as asthma, atopic dermatitis and nasal allergies, and reactions to allergens and environmental pollutants. All these have led to the notion that agents or methods protecting and maintaining functionality of the epithelial barrier could be beneficial for prevention or attenuation of these conditions.

While considering the two drug delivery routes, the nasal and oral drug delivery, the nasal delivery route has some apparent advantages. As has been noted, nasal delivery permits to avoid hepatic first-pass metabolism. The nasal cavity has a relatively large surface area and its epithelium and sub-mucosal are characterized by high vascularization and high substance permeability, all of which promote rapid drug absorption. In addition, it offers a convenient option of self-medication.

The mucoadhesive polymers, and especially hydrophilic biopolymers and polysaccharides from natural sources, are attracting increasing attention as vehicles and actives for oral and nasal drug delivery. One example is carrageenan, a natural sulfated polysaccharide extracted from red algae, which has been related to antiviral properties. At the same time, carrageenan per se has proved to be highly inflammatory and to perform poorly in terms of spray coverage and mucoadhesion. To overcome these problems, carrageenan is usually used in a mixture with other biocompatible polysaccharides such as gellan gum ³. Another algal polysaccharide is alginate, with certain examples of mucoadhesive drug delivery systems using thereof as a carrier for conventional drugs ⁴. Certain hybrid mucoadhesive delivery systems using a polymer with drug-loaded lipid nanoparticles were described in US201922411 and WO19246384 with systems adapted for personalized dosing.

Overall, despite the apparent advantages of mucoadhesive polymers, there are still some concerns about their toxicity, even with biocompatible mucoadhesive polymers, as well as with their mucoadhesion and spread and coverage properties. From a broader perspective, there is an unmet need to find solutions for effective and safe systemic drug delivery, and specifically delivery of drugs related to the restoration and maintenance of functionality and intactness of the oral and nasal epithelial mucosa barrier. The worldwide prevalence of COVID-19 and conditions such as allergies and air pollution damages ultimately reinforce the need to provide such solutions.

### References

1. Zou L et al. SARS-CoV-2 viral load in upper respiratory specimens of infected patients. N. Engl. J. Med. 382(12), 1177-1179 (2020).
2. Hamming I et al. Tissue distribution of ACE2 protein, the functional receptor for SARS coronavirus. A first step in understanding SARS pathogenesis. J. Pathol. 203(2), 631-637 (2004).
3. Robinson TE et al. Low acyl gellan as an excipient to improve the sprayability and mucoadhesion of iota carrageenan in a nasal spray to prevent infection with SARS-COV-2. Front. Med. Tech. 3, article 687681 (2021).
4. Patil SB and Sawant KK. Development, optimization and in vitro evaluation of alginate mucoadhesive microspheres of carvedilol for nasal delivery. J. Microencapsul. 26(5): 432-443 (2009).

### GENERAL DESCRIPTION

Epithelial barrier is the first line defense against exposure to harmful pathogens, allergens, and other foreign particles. The intactness of the epithelial tissue or the epithelial mucosa plays a critical role in both innate and adaptive mucosal immunity. Epithelial cells are responsible for activation of functional molecules (e.g., pro-inflammatory cytokines, growth factors and chemokines), and secretion of antimicrobial substances and peptides (AMPs, e.g., lysozyme, defensins, lactoferrin, S-100 proteins) that interfere with the entry of pathogens. Mucociliary clearance is responsible for trapping of microbes and particles in the mucus secreted by glands and ciliated cells in the oral and nasal cavities. Altered homeostasis of any of these systems play an important role in inflammatory and infectious diseases in the upper airways.

The nasal epithelial barrier is further characterized by apical junctional complexes (AJCs) allowing interconnections between epithelial cells (TJs, adherens junctions, desmosomes, hemidesmosomes). TJs impairment is the primary cause in the pathogenesis of allergic rhinitis (AR), for example, a common disorder affecting up to 40% of the population worldwide and persisting throughout life. Additional examples are asthma, atopic dermatitis, nasal allergies, and reactions to allergens and environmental pollutants.

In COVID-19 infection, the virus enters mainly through the nasal cavity, where it grows and destroys cells of the nasal mucosa thereby allowing systemic spread of the virus and triggering of local and systemic inflammation processes. With the appearance of symptoms, the disease pathophysiology has been already established. And if the infection and inflammation persist, they can lead to a secondary full-blown disease with a respiratory failure, systemic shock and potentially multi-organ failure.

Therefore, to effectively address infections, allergies and disorders of the upper airways, a more straightforward approach would be to prevent or obstruct the contact or entry of the causative agent into the nasal or oral cavities, thereby minimizing the risk of subsequent infection or allergic response. For viruses, and for COVID 19 in particular, an important objective would be to minimize the viral load in the nasal cavity to prevent its transmission to other individuals and spread in the general population.

Mucoadhesive agents per se or in combination with antiviral, anti-inflammatory or antiallergic drugs, are attractive candidates for such preventive approaches in offering a convenient and economic method of protection against pathogens and allergens that can be easily applied on individual and population levels.

Within this framework, the present invention provides particularly advantageous biocompatible mucoadhesive compositions, which when applied onto a cellular or a mucosal surface become transformed into a continuous, durable and non-toxic film that can serve as an effective protective barrier against a variety of pathogens, allergens and air pollutants. The validity and efficacy of this protection has been corroborated by a series of experiments in various models of viral infection and allergen challenge.

In other words, the compositions of the invention per se, without added actives, proved to be effective and safe mucoprotective agents for two highly prevalent conditions - a viral infection and an allergen challenge. More generally, due to their accessibility, biocompatibility and capacity to incorporate of additional therapeutic actives, they can provide a safe and straightforward solution for prevention both, transmission of pathogens and excessive exposure to harmful pollutants, on individual and populational levels.

More specifically, the invention provides compositions comprising combinations of sulfated polysaccharides and hydrophobic or hydrophilic polymers that are partially crosslinked by di-valent cations. This specific composition of components has been presently demonstrated to possess unique and surprising physical properties of an improved gel/liquid consistency, rheological behavior and transformability into a film, and further, surprising biological properties of effective mucoadhesivity, mucoprotectivity and general lack of toxicity.

Starting from the physical properties, the present compositions have been shown to possess a substantially constant viscosity at room temperature (RT) in terms of a viscosity as measured by a viscometer and rheological behavior under shear forces. The viscosity of these compositions was further lower (up to 20%) than the viscosity of analogous solutions with the same concentrations of polymers but without the partial crosslinking. The rheological behavior (or viscosity behavior) of these compositions under varying shear forces was also surprisingly constant and was substantially more constant (<25% deviation) than the behavior of analogous non-crosslinked solutions measured under the same conditions **(EXAMPLE 5).**

These two core physical properties, the lower and constant viscosity and the relative resistance to varying shear forces at RT, are responsible for the main structural advantages of the present compositions - an improved gel/liquid consistency or spread-ability/spray-ability when presented alone, and an ability to form or a transformability into a continuous and durable film upon their contact with cellular surfaces.

The intactness, durability and the protectiveness of this film have been proven in a series of experiments in various models of viral infection and allergen challenge in vitro. Owing to this film-forming ability, the present compositions proved to be very effective mucoprotective agents against various types of viruses, including several types of corona viruses and influenza virus **(EXAMPLES 2-3).** They were also found effective against a relatively broad range of aeroallergens, including a number of common determinants of allergic reaction and allergen related inflammation. In the context of allergen challenge, moreover, compared to the other tested compositions (the non-crosslinked solutions and single polymer compositions) their protective effect was substantially more significant and synergistic **(EXAMPLE 4)** In addition, they were demonstrated to be non-toxic over a wide range of pharmaceutically applicable concentrations **(EXAMPLE 1).**

All these properties make the present compositions especially suitable for topical and mucosal applications, and specifically for applications onto oral and/or nasal mucosa.

More generally, the invention forms the basis for two types of products:
(1) OTC products using the compositions per se or in combination with OTC approved actives (Xylometazoline, Ibuprofen, decongestants, etc.) to provide safe and effective coverage of mucosal tissues (e.g., oral and/or nasal mucosa) against exposure to pathogens, allergens and other air-born agents.
(2) Prescription based medications and methods using the compositions in combination with conventional drugs to treat an already existing infection and/or inflammation, and in case of viruses, to mitigate its transmission to other hosts.

Both types of products can be provided in a variety of convenient forms, for example as sprays or microparticles for inhalation for oral or nasal applications.

One advantage of these compositions stems from the biocompatibility and accessibility of their core components, the sulfated polysaccharides, hydrophobic and hydrophilic polymers and di-valent cations. A variety of such polymers can be obtained from natural sources, plants, animal and microorganisms. Notable examples are sulfated polysaccharides produced by various types of marine algae, such as galactans, ulvans, fucans and fucoidans, with specific examples of agaran and carrageenan galactans. Examples of biocompatible hydrophilic polymers are dextrans, alginates, chitosans, agaroses, pullulans polysaccharides, and albumins, gelatins, collagens and lectins proteins, all of which are obtainable from natural sources. Examples of crosslinking di-valent cations are Ba²⁺, Ca²⁺, Co²⁺, Cu²⁺, Fe²⁺, Mg²⁺ and Zn²⁺.

More specific examples are compositions comprising combinations of carrageenan and alginate partially crosslinked by Ca²⁺ in concentrations ranges of about 0.005%-1%, about 0.1%-3%, about 0.0001%-1% (w/w), respectively.

The compositions can further comprise buffers, excipients, preservatives, flavoring and odor agent so as to be compatible with and optimally adapted for coverage by spraying or administration by inhalation via oral and/or nasal routes.

As pharmaceutical compositions, the compositions can further incorporate conventional therapeutic actives, as per specific clinical indication. Attractive candidates can be drugs for treating a widely prevalent group of sub-clinical and clinical conditions commonly referred to as oral, nasal or upper airway mucosal barrier dysfunction.

In the broadest sense, the invention applies to any condition related to an altered homeostasis of the epithelial or mucosal barrier or loss of its intactness and functionality. It can further apply to a condition related to an impairment of physical continuity, cellular makeup, functionality of mediators of innate and adaptive immunity, and other functions in the epithelial milieu. Specific examples of such conditions are microbial (viral and/or bacterial) infections, and aeroallergen induced allergies and inflammations, for which the present compositions alone or in combination other anti-viral, antibiotic and/or anti-inflammatory drugs can provide effective and safe protection.

From yet another perspective, the compositions of the invention provide new drug delivery systems for introducing systemic drugs via administration onto various mucosal tissues. So far, the most common routes for systemic drug administration are oral, enteral or parenteral routes, with their specific merits and disadvantages. The invention provides an alternative route of drug administration via mucoadhesion and permeation through various mucosal tissues, including genitourinary, rectal, oral and nasal mucosa, lung and other mucosa.

This aspect can further involve additional technologies permitting targeted and/or controlled release of actives, either to the epithelial or systemic milieu, including micro- and nanonization and encapsulation technologies to improve drug safety and efficacy.

The presently proposed approach can be especially useful for formulation of highly lipophilic actives. About 60%-70% of known drugs, and even a higher proportion of modern drug candidates, are highly lipophilic. Lipophilic actives generally suffer from low oral bioavailability, poor gastrointestinal (GI) permeability and absorption, GI efflux and hepatic first-pass metabolism. The epithelial mucosa, and specifically the oral and nasal mucosa, is inherently rich in vascularization and have relatively high permeability, independent of the hepatic first-pass metabolism. Therefore, lipophilic drugs using these routes are likely to have a better drug bioavailability and absorption. Solubility and bioavailability can be further improved by incorporation of specific permeation enhancers, solubilizers, emulsifiers and surfactants into the present compositions.

In other words, due to their superior properties of mucoadhesivity, coverage and protectivity, the present compositions may have better characteristics of drug retention, and as a result, improved absorption and assimilation of the drug onto mucosal tissues, which can be further enhanced by additional formulation, nanonization and encapsulation technologies and use of patches or films for localized and systemic drug delivery.

One interesting application in the context of local or systemic delivery can be oral and/or nasal delivery of vaccines, including antiviral vaccines and vaccines for other disorders. Being relatively non-invasive and "needle free", such vaccines can be easily incorporated into various preventive strategies on individual- and population-basis.

Yet another interesting application of the present compositions is in tissue regeneration and bioengineering of tissue implants. Owing to their biocompatibility and physical, chemical and other properties, these compositions can serve as a universal "fits all" material in the form of matrixes and/or injectable gels to provide physical support for regeneration of tissues and organs and to support cell assembly at specific sites. Adaptability of the present compositions to incorporate additional materials, actives and drugs makes them attractive candidates for this application.

For example, the matrixes/gels made of these compositions can further include actives, drugs, growth factors, hormones, enzymes, nucleic acids and other agents to provide morphological and biochemical cues for specific cell assembly, growth and functionality. Depending on the required indication, the matrixes/gels can further include encapsulated cells of various types and origins. Specific examples are various types of stem cells that upon appropriate triggering can lead to assembly, proliferation and/or differentiation of cells at specific application sites.

In the neuronal or cardiac context, the present compositions can assist in structural and functional restoration of damaged tissues by delivering mechanical and functional support for altering the cell fate in favor of neuronal or cardiac regeneration, leading to repair of infarcted heart, injured spinal cord or a degenerative brain tissue. When viewed as tools for neurorepair or regeneration, the present compositions can be combined with assisting devices and incorporated into neurological surgical procedures.

Ultimately, the invention provides a wide range of products, methods and uses based on biocompatible mucoadhesive compositions in the form of sprays, gels, matrixes or films, used either independently or in conjunction with other drugs or delivery devices, as mucoprotective antiviral and/or anti-inflammatory agents, agents assisting in tissue regeneration and repair, and local and systemic drug delivery.

The following numbered clauses show further illustrative examples only:
**1.** A composition for delivery onto an epithelial mucosa, the composition comprises at least one sulfated polysaccharide polymer and at least one hydrophobic or hydrophilic polymer that are partially crosslinked by at least one positively charged ion,
   the composition has a substantially constant viscosity at room temperature (RT), whereby upon contact with the epithelial mucosa the composition is transformable into a uniform and continuous film.
**2.** The composition of clause **1,** wherein the epithelial mucosa is oral and/or nasal mucosa.
**3.** The composition of clause 1, wherein before contact with the epithelial mucosa, the composition is in a liquid, a semiliquid or sprayable form.
**4.** The composition of clause **1,** wherein the substantially constant viscosity is a viscosity that resides within a range between about 1 mPa*s⁻¹ and about 20 mPa*s⁻¹ for a period of at least 10 days or more, as measured by a viscometer.
**5.** The composition of clause **4,** wherein the viscosity is up to 20% lower than the viscosity of a solution comprising the same concentrations of the same polymers but no positively charged ions when measured under the same conditions.
**6.** The composition of clause **1,** wherein the substantially constant viscosity is a viscosity measured under varying shear forces and resides within a range between about 100 mPa*s⁻¹ and about 800 mPa*s⁻¹ for a shear rate in the range between about 0.1 and about 10 s⁻¹, as measured by a rheometer.
**7.** The composition of clauses **1** or **6,** wherein the substantially constant viscosity is a viscosity measured under varying shear forces and has less than 25% deviation for varying shear rates in the range between about 0.1 and about 10 s⁻¹
**8.** The composition of clause **1,** comprising micro or nano particles or droplets.
**9.** The composition of clause **1,** wherein the at least one sulfated polysaccharide polymer and the at least one hydrophobic or hydrophilic polymer are synthetic, semisynthetic or modified natural polymers, or a combination thereof.
**10.** The composition of clause **1,** wherein the at least one sulfated polysaccharide polymer and the at least one hydrophobic or hydrophilic polymer are natural polymers obtained from a plant, an animal or a microbial origin, or a combination thereof.
**11.** The composition of clause **1,** wherein the at least one sulfated polysaccharide polymer is selected from sulfated galactans, ulvans, fucan, fucoidans, heparins, glucosamines, dextrans, chitosan, chitin and chondroitin.
**12.** The composition of clause **1,** wherein the at least one sulfated polysaccharide polymer is at least one carrageenan selected from iota carrageenan, kappa carrageenan and lambda carrageenan.
**13.** The composition of clause **1,** wherein the at least one hydrophilic polymer is a polysaccharide or a protein.
**14.** The composition of clause **1,** wherein the at least one hydrophilic polymer is a polysaccharide selected from dextrans, alginates, chitosans, agaroses and pullulans.
**15.** The composition of clause **1,** wherein the at least one hydrophilic polymer is a protein is selected from albumins, gelatins, collagens, lectins, legumines, and vicilines.
**16.** The composition of clause **1,** wherein the at least one hydrophilic polymer is alginate or an alginate salt.
**17.** The composition of clause **1,** wherein the at least one positively charged ion is a di-valent cation selected from Ba²⁺, Be²⁺, Ca²⁺, Co²⁺, Mg²⁺, Cu²⁺, Ni²⁺, Fe²⁺ and Zn²⁺.
**18.** The composition of clause **1,** wherein the at least one sulfated polysaccharide polymer is a carrageenan and the at least one hydrophilic polymer is an alginate and the at least one positively charged ion is Ca²⁺.
**19.** The composition of clause **18,** wherein the carrageenan is at a concentration in a range between about 0.005% and about 1%, the alginate is at a concentration in a range between about 0.1% and about 3%, and the Ca²⁺ is at a concentration in a range between about 0.0001% and about 1% (w/w).
**20.** The composition of clause **19,** wherein the carrageenan is at a concentration in a range between about 0.1% and about 0.3%, the alginate is at a concentration in a range between about 1% and about 3%, and the Ca²⁺ is at a concentration in a range between about 0.0001% and about 0.05% (w/w).
**21.** The composition of clause **19,** wherein the carrageenan is at a concentration in a range between about 0.1% and about 0.3%, the alginate is at a concentration in a range between about 2.0% and about 2.9%, and the Ca²⁺ is at a concentration in a range between about 0.005% and about 0.02% (w/w).
**22.** The composition of clause **19,** wherein the carrageenan is at a concentration in a range between about 0.1% and about 0.3%, the alginate is at a concentration in a range between about 2.0% and about 2.9%, and the Ca²⁺ is at a concentration in a range between about 0.0001% and about 0.005% (w/w).
**23.** The composition of any one of the preceding clauses further comprising a solvent, a buffer, an excipient, an adjuvant, a permeation enhancer, a plasticizer, a thickener, a rheological modifier, an alcohol, an extender, a preservative, a colorant, a pigment, a sweetener, a flavoring or an odor agent, or combinations thereof.
**24.** The composition of any one of the preceding clauses further comprising at least one additional therapeutic agent.
**25.** A pharmaceutical composition comprising a therapeutically effective amount of the composition of any one of clauses **1** to **24** and a pharmaceutically acceptable buffer, carrier or excipient.
**26.** The pharmaceutical composition of clause **25,** wherein the pharmaceutical composition is adapted for inhalation in the form of particles or droplets.
**27.** The pharmaceutical composition of clause **26,** the pharmaceutical composition being adapted for oral and/or nasal administering in the form of a spray or a gel.
**28.** The pharmaceutical composition of any one of clauses **25** to **27,** further comprising at least one additional therapeutic agent for preventing or treating a disorder related to oral and/or nasal mucosal barrier dysfunction.
**29.** A kit comprising the composition of clauses **1** or **24** and a device for oral or nasal delivery or inhalation, and further comprising instructions for use.
**30.** A drug delivery system comprising the composition of any one of clauses **1** to **24** and at least one additional therapeutic agent.
**31.** The drug delivery system of clause **30,** wherein the at least one additional therapeutic agent is an agent for preventing or treating a disorder or condition related to oral and/or nasal mucosal barrier dysfunction.
**32.** The drug delivery system of clause **30,** wherein the at least one additional therapeutic agent is an agent for preventing or treating a clinical disorder or a clinical or subclinical condition.
**33.** The drug delivery system of clauses **31** or **32,** wherein the at least one additional therapeutic agent is selected from antibiotics, antifungal agents, antiviral agents, neuroleptics, analgesics, hormones, anti-inflammatory drugs, non-steroidal anti-inflammatory drugs, anti-rheumatic, drugs anticoagulants, beta-blockers, diuretics, anti-hypertension drugs, anti-atherosclerosis drugs, antidiabetics, anti-asthmatic drugs, decongestant and/or cold medicines.
**34.** A method for drug delivery, the method comprises an oral and/or a nasal administering of the composition of any one of clauses **1** to **24,** the pharmaceutical composition of any one of clauses **25** to **28** or the drug delivery system of any one of clauses **30** to **33.**
**35.** A composition of any one of clauses **1** to **24** for use in protecting oral and nasal mucosal barrier function.
**36.** A composition of any one of clauses **1** to **24** for use in preventing, alleviating or treating a disorder or a condition related to oral and/or nasal mucosal barrier dysfunction.
**37.** A method for protecting oral and/or nasal mucosal barrier function in a subject, the method comprises oral or nasal administering to the subject a therapeutically effective amount of the composition of any one of clauses **1** to **24.**
**38.** The method of clause **37,** further comprising administering to the subject at least one additional therapeutic agent.
**39.** A composition of any one of clauses **1** to **24** for use in treating, alleviating and/or preventing a microbial oral and/or nasal infection.
**40.** The composition of clause **39,** wherein the microbial oral and/or nasal infection is a viral and/or a bacterial infection.
**41.** The composition of clause **40,** further comprising at least one antibiotic and/or antiviral drug.
**42.** The composition of clause **41,** wherein the antiviral drug is an antiviral vaccine.
**43.** A composition of any one of clauses **1** to **24** for use in treating, alleviating and/or preventing an aeroallergen induced allergy and/or inflammation.
**44.** The composition of clause **43,** further comprising at least one anti-allergic and/or anti-inflammatory drug.
**45.** A method for treating, alleviating, and preventing a microbial oral and/or nasal infection in a subject, the method comprises oral and/or nasal administering to the subject a therapeutically effective amount of a composition of any one of clauses **1** to **24.**
**46.** The method of clause **45,** further comprising concomitant administering to the subject at least one antibiotic and/or antiviral drug, said administering being via oral, enteral, parenteral, or nasal routes.
**47.** A method for preventing, alleviating or treating an aeroallergen induced allergy and/or inflammation in a subject, the method comprises oral and/or nasal administering to the subject a therapeutically effective amount of a composition of any one of clauses **1** to **24.**
**48.** The method of clause **47,** further comprising concomitant administering to the subject at least one anti-allergic and/or anti-inflammatory drug, said administering being via oral, enteral, parenteral, or nasal routes.
**49.** A composition of any one of clauses **1** to **24** for use in regeneration of a tissue and/or in bioengineering a tissue implant.
**50.** The composition of clause **49,** wherein the tissue is a neuronal or a cardiac tissue and/or the tissue implant is implanted into a neuronal or a cardiac tissue.
**51.** A method of regeneration of a tissue and/or bioengineering a tissue implant in a subject in need thereof, the method comprises administering to the tissue or implanting to the tissue of said subject the composition of any one of clauses **1** to **24.**
**52.** The method of clause **51,** wherein the tissue is a neuronal or a cardiac tissue and/or the tissue implant is implanted into a neuronal or a cardiac tissue.
**53.** The method of clause **51,** wherein said administering or implanting further comprises a surgical procedure.
**54.** Use of the composition of any one of clauses **1** to **24** for the manufacture of a medicament for preventing, alleviating or treating an aeroallergen induced allergy and/or inflammation.
**55.** Use of the composition of any one of clauses **1** to **24** for the manufacture of a medicament for preventing, alleviating or treating a microbial oral and/or nasal infection.
**56.** Use of a composition of any one of clauses **1** to **24** for the manufacture of a medicament for preventing, alleviating or treating a clinical disorder or a clinical or subclinical condition.
**57.** Use of a composition of any one of clauses **1** to **24** for the manufacture of a medicament for preventing, alleviating or treating a disorder or a condition related to oral and/or nasal mucosal barrier dysfunction.

### BRIEF DESCRIPTION OF THE DRAWINGS

To better understand the subject matter and to exemplify how it may be carried out in practice, embodiments will now be described by way of non-limiting examples with reference to the following drawings.

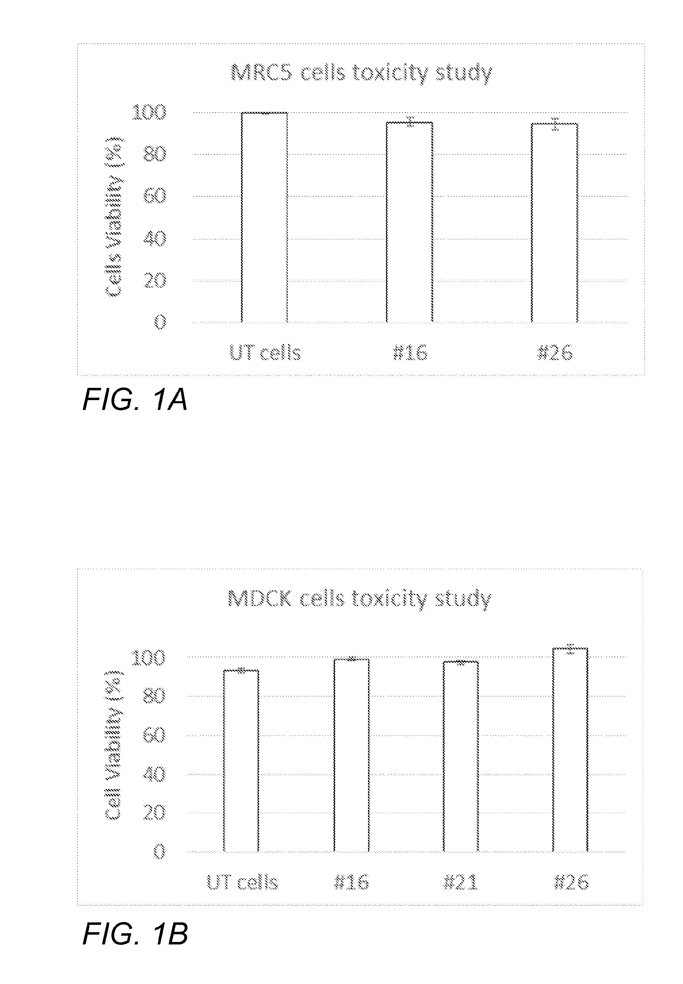

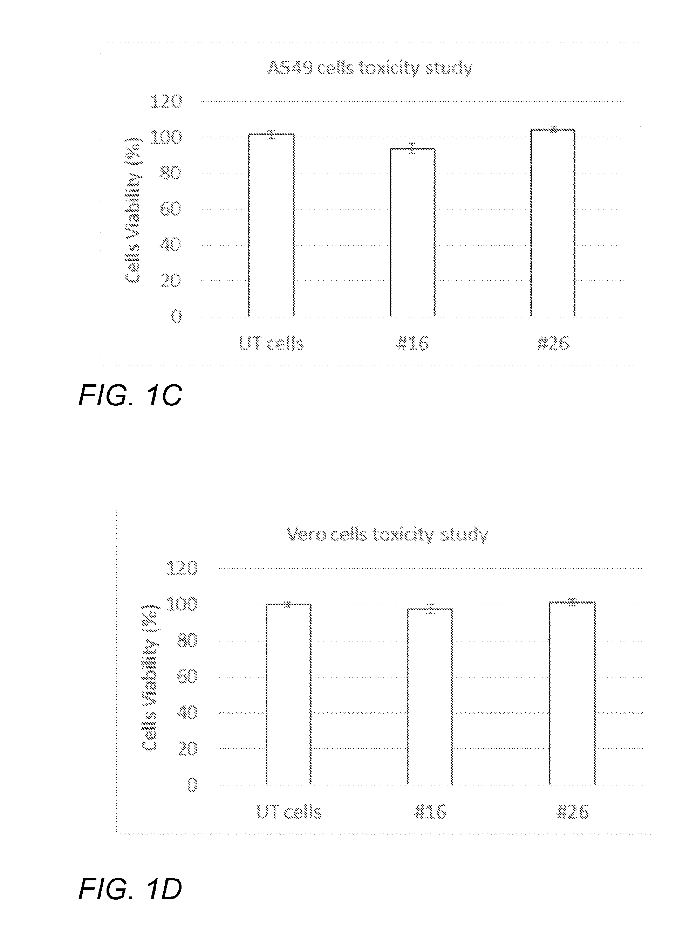

**Figs 1A-1D** illustrate the lack of cytotoxicity (safety) of the present compositions, comparing the effect of compositions with the alginate/carrageenan combination in non-crosslinked (#16) and partially crosslinked (#21, #26 with 1.47%, 0.02% CaCl₂ respectively) forms vs. untreated controls (UT) in MRC-5 **(A),** MDCK **(B),** A549 **(C)** and Vero **(D)** cells. No cytotoxicity was observed in any of the tested models.

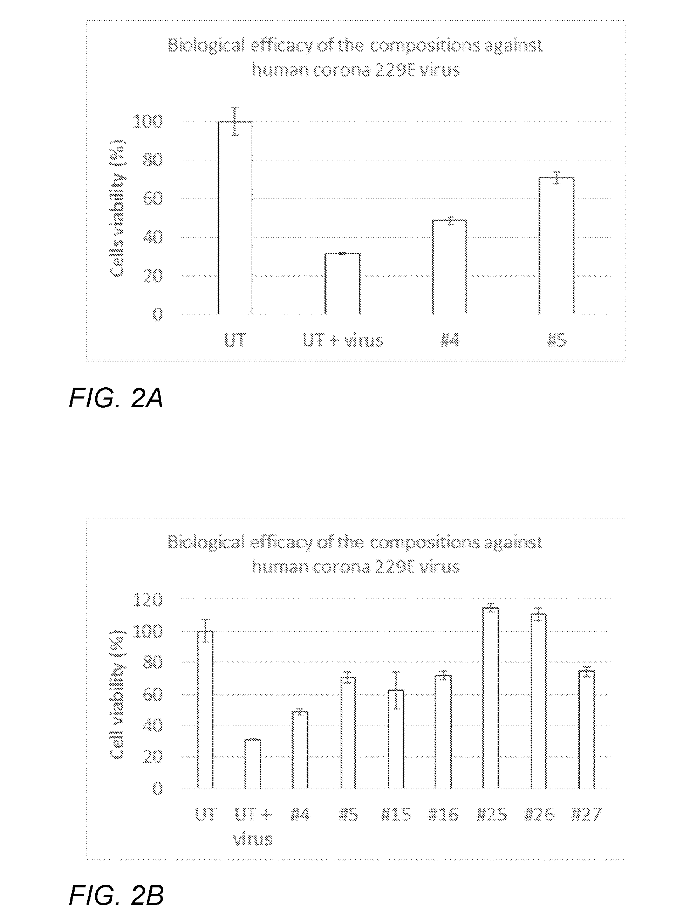

**Figs 2A-2B** illustrate the antiviral mucoprotective effect of the present compositions in a model of human corona virus (229E) in vitro, comparing the effect of compositions with single polymers (#4 alginate, #5 carrageenan) **(A)** and **(B),** and compositions with the alginate/carrageenan combination in non-crosslinked (#15, #16 carrageenan 0.12% and 0.24%, respectively) and partially crosslinked (#25, #26 carrageenan 0.12% and 0.24%, respectively) forms and partially crosslinked alginate (#27) **(B)** vs. untreated cells (UT) and cells exposed to the virus (UT+virus). The compositions with the partially crosslinked polymers were significantly more effective against 229E than the compositions with single and non-crosslinked polymers.

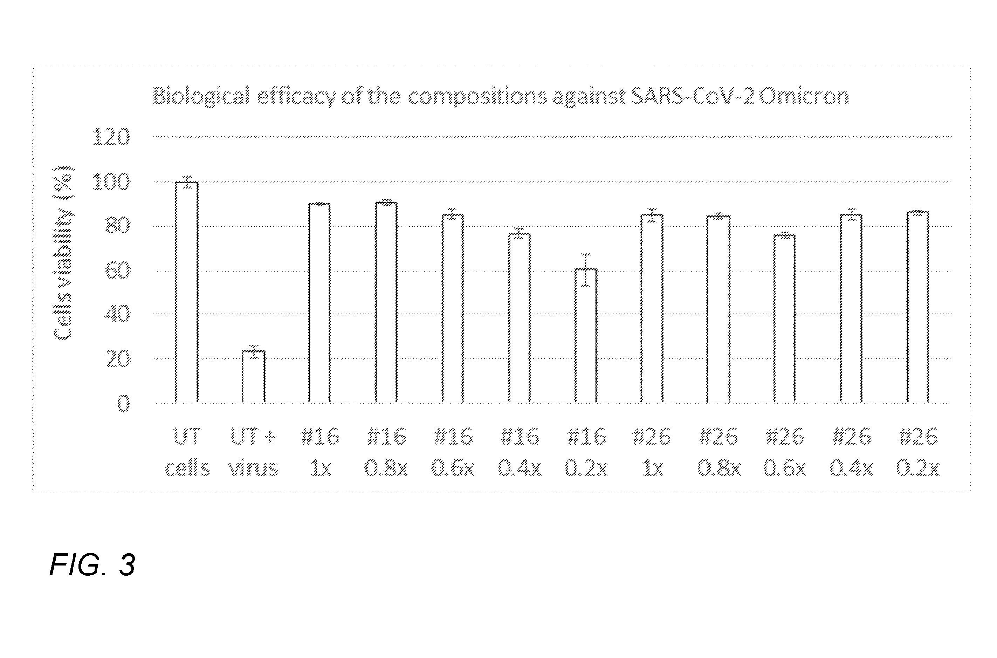

**Fig. 3** illustrates the antiviral mucoprotective effect of the compositions on another corona virus, SARS-CoV-2 (Omicron variant, B.1.1.529.BA.1), comparing the effect of compositions with non-crosslinked (#16) and partially crosslinked (#26) polymers in various concentrations (x1, x0.8, x0.6, x0.4, x0.2) vs. UT and UT+virus controls. The compositions with the partially crosslinked polymers were more effective against SARS-CoV-2, including in the lower concentrations of polymers (x0.2).

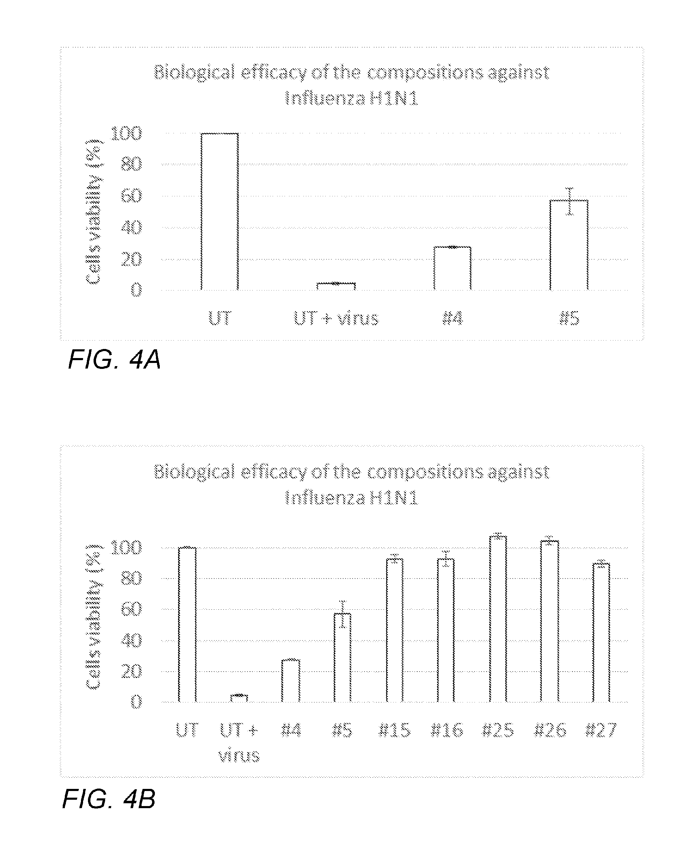

**Figs 4A-4B** illustrate the antiviral mucoprotective effect of the compositions on human influenza virus (H1N1), comparing the effect of compositions with single non-crosslinked polymers (#4, #5) **(A)** and **(B),** and compositions with non-crosslinked (#15, #16) and partially crosslinked (#25, #26) combination of polymers and partially crosslinked alginate (#27) **(B)** vs. UT and UT+virus controls. The compositions with the partially crosslinked polymers proved to be equally effective with H1N1, and more effective than the non-crosslinked and other compositions.

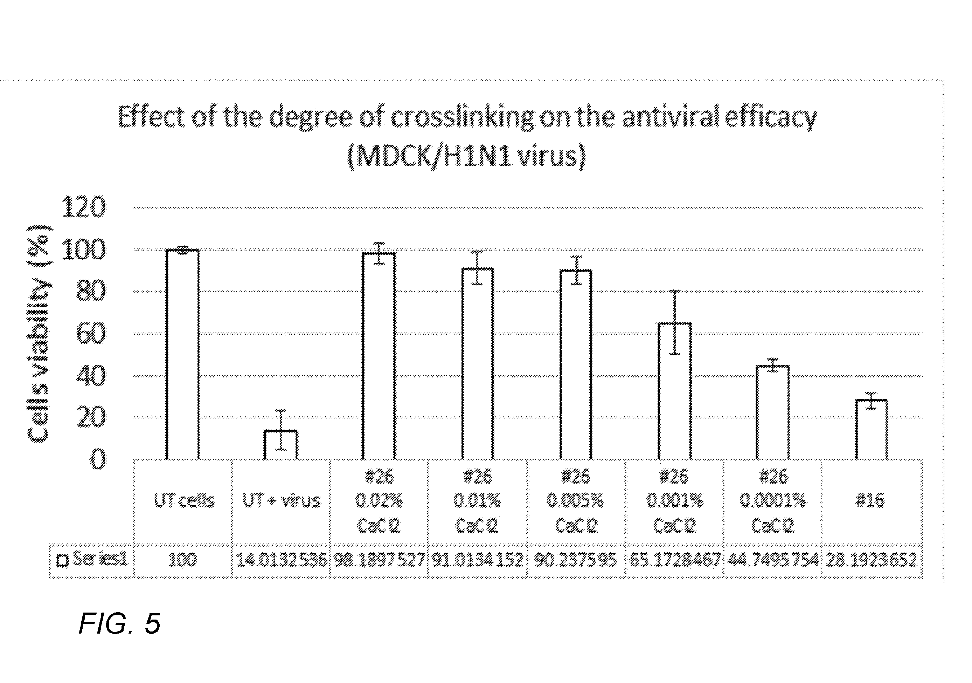

**Fig. 5** illustrates the effect of a degree of crosslinking in the same H1N1 model, comparing the effect of compositions with alginate-carrageenan polymers with various degrees of crosslinking (#26 with 0.0.2%, 0.01%, 0.005%, 0.001%, 0.0001% CaCl₂ and non-crosslinked polymers (#16) and vs. UT and UT+virus controls. The compositions with the combination of polymers and crosslinking by CaCl₂ in the range of 0.005%-0.02% had the highest antiviral mucoprotective effect (>90% cell viability), and in the range of 0.0001%-0.02% still had a higher effect than the non-crosslinked compositions.

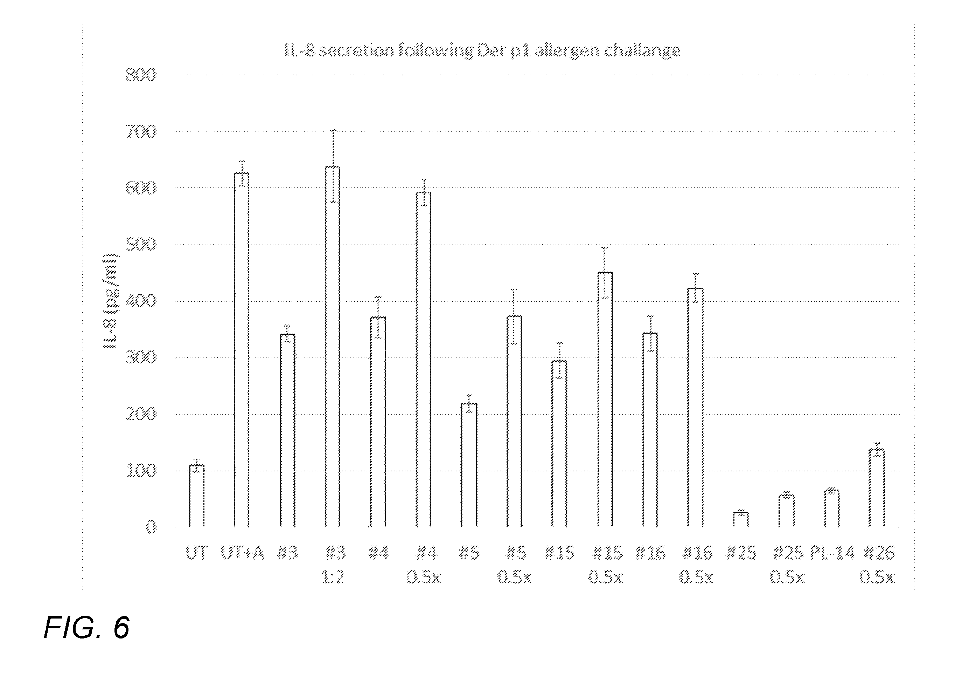

**Fig. 6** illustrates the anti-inflammatory mucoprotective effect of compositions in a model of allergen challenge in vitro using dust mite Der p1 allergen and IL-8 marker, comparing the effect of compositions with single polymers (#3 iota carrageenan, #4 alginate, #5 carrageenan) and with non-crosslinked (#15, #16) and partially crosslinked (#25, #26) alginate/carrageenan, in two concentrations (x1, x0.5) vs. UT and UT+virus controls. In this model, the compositions with the partially crosslinked combination of polymers demonstrated a synergistic effect compared to the non-crosslinked and single polymer compositions.

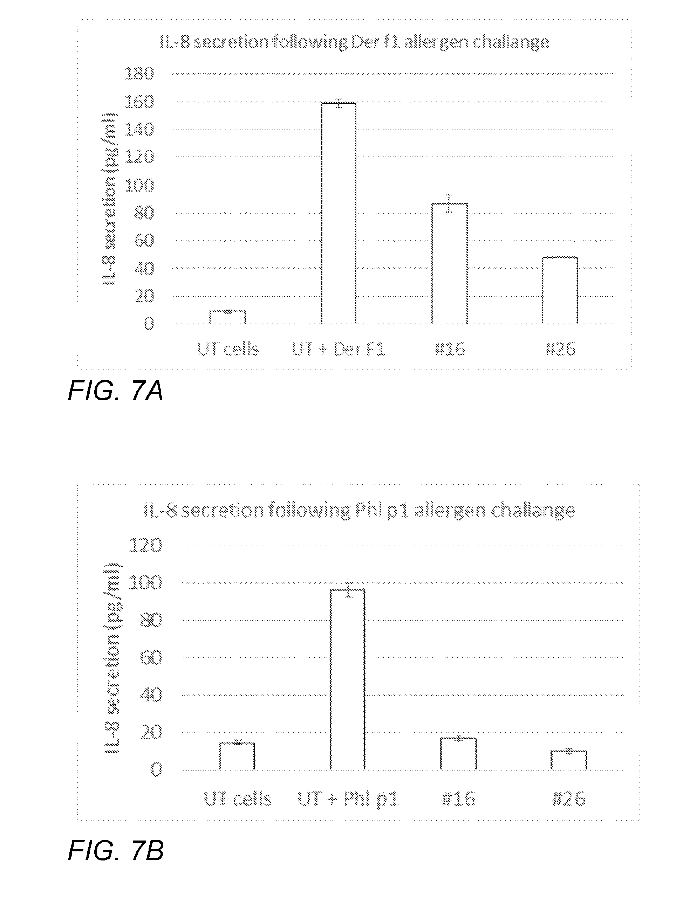

**Figs 7A-7B** illustrate the anti-inflammatory mucoprotective effect on additional allergens, a dust mite allergen Der f1 and a pollen allergen Phl p1, comparing the effect of compositions with non-crosslinked (#16) and partially crosslinked (#26) polymers vs. UT and UT+virus controls. Also in this model, the composition with partially crosslinked combination of polymers was found to be more effective.

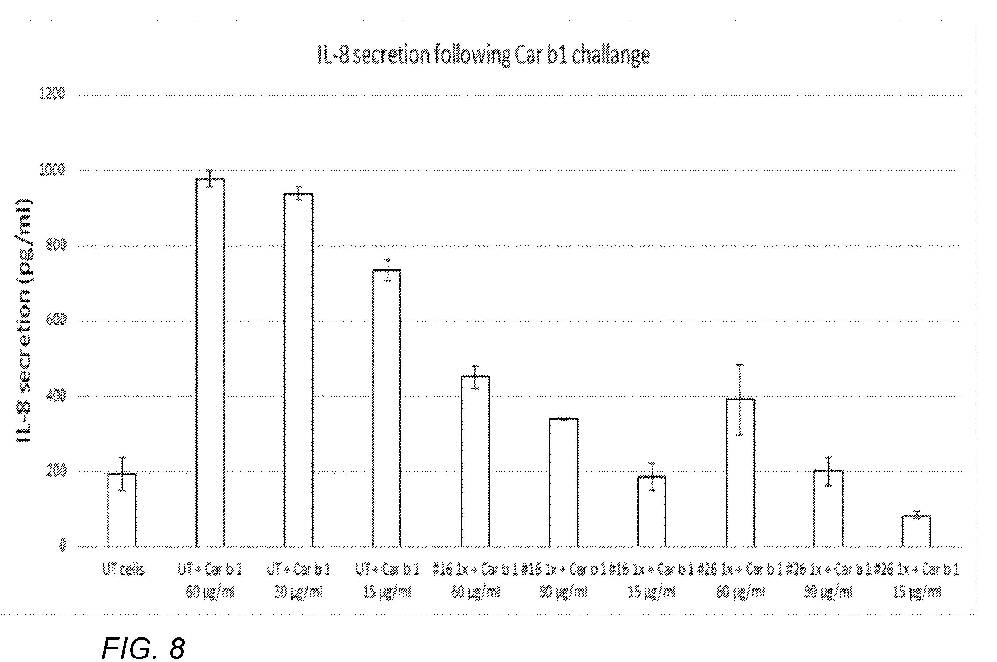

**Fig. 8** illustrates the same effect on another pollen allergen, Car b1, using the same experimental model and various concentrations of Car b1 (60 µg/ml, 30 µg/ml, 15 µg/ml). In all tested groups, the effect was consistent with a classical dose-response, with the compositions with the partially crosslinked polymers showing the highest anti-inflammatory mucoprotective effect (the lowest IL-8).

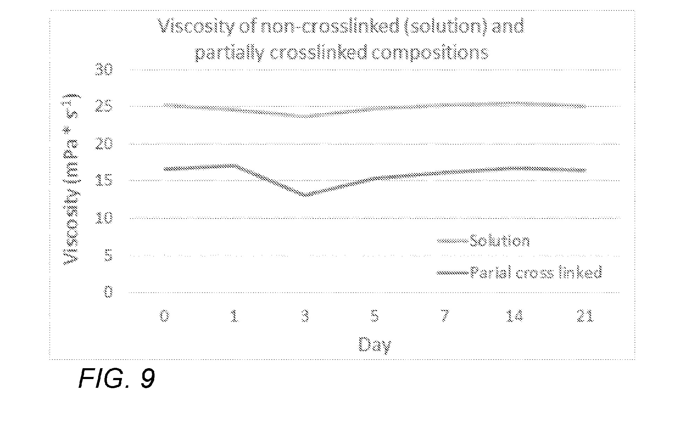

**Fig. 9** illustrates one of the distinguishing properties of the present compositions revealed in a lower viscosity and viscosity sustainability at RT, comparing the viscosity (25°C) of the compositions with the non-crosslinked (#16) and partially crosslinked (#26) polymers over a prolonged period (21 days). While both types of compositions had a relatively constant viscosity at RT over the tested period, the partially crosslinked composition had a lower viscosity, suggesting a better spray-ability and coverage and transformability into a spreadable, uniform and continuous film.

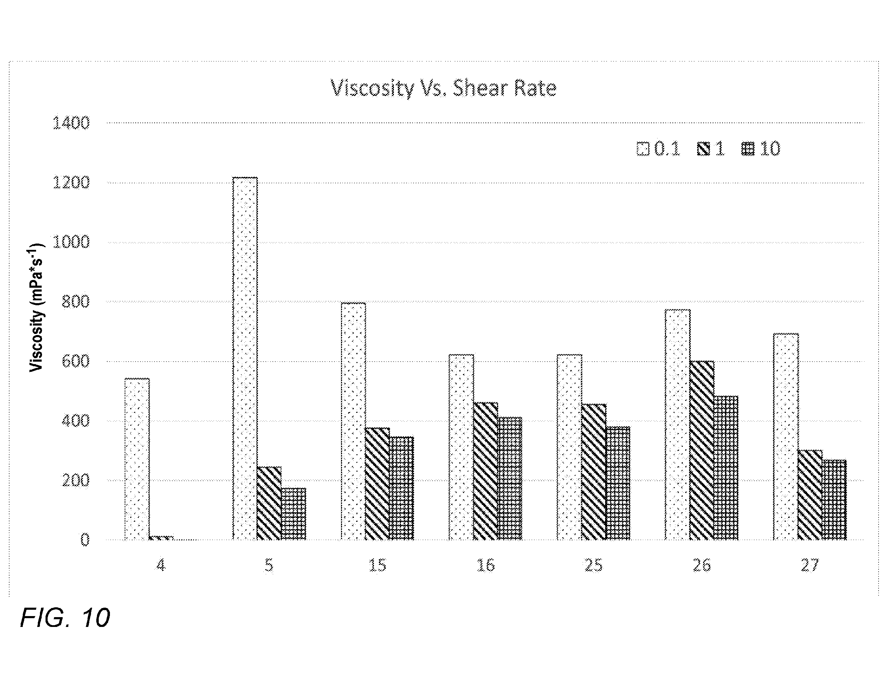

**Fig. 10** illustrates the property of a sustained or constant viscosity under varying shear forces, comparing the viscosity of compositions with single (#4 alginate, #5 carrageenan) and combination of non-crosslinked (#15) and partially crosslinked (#25, #26) polymers and partially crosslinked alginate (#27) under varying shear rates (0.1, 1 and 10 s⁻¹). The viscosity of compositions with partially crosslinked polymers was resistant to shear forces, indicative of their transformability into a uniform and durable film at RT.

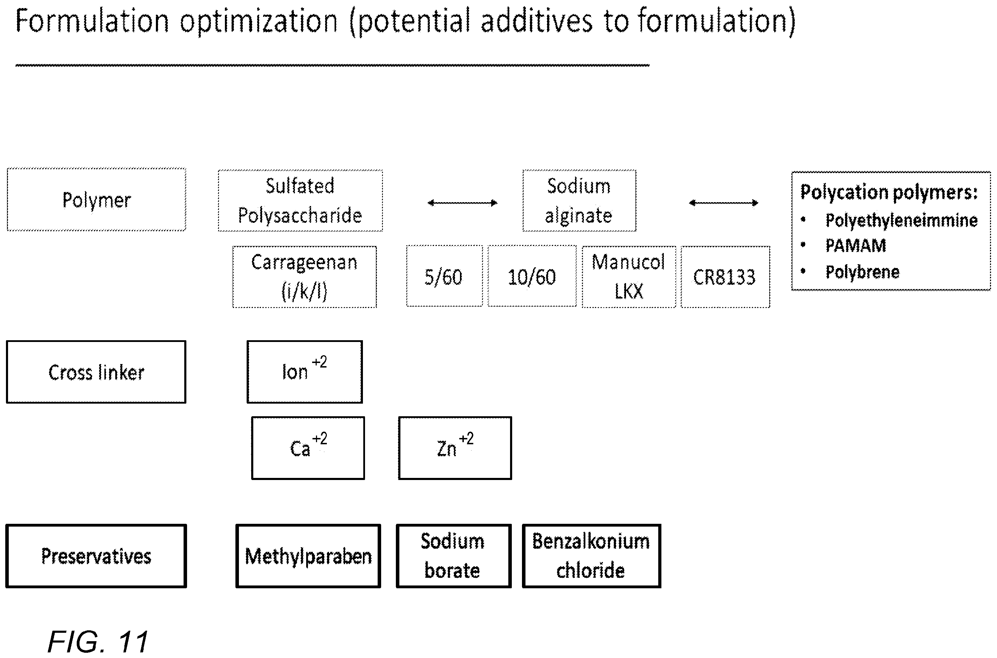

**Fig. 11** is a schematic illustration of additional embodiments of the compositions comprising optional polymers, crosslinkers and preservatives.

### DETAILED DESCRIPTION OF EMBODIMENTS

It should be noted that the invention is not limited to specific methods, and experimental conditions described herein, and that the terminology used herein serves only illustrative purposes and is not intended to be limiting.

In the broadest sense, the present invention provides compositions comprising a composite material comprising one or more sulfated polysaccharide polymers and one or more hydrophobic or hydrophilic polymers or copolymers that are partially crosslinked by one or more positively charged ions.

The term "***sulfated polysaccharide polymer"*** broadly refers herein to a group of compounds generally characterized by a negatively charged large chain of simple sugars covalently linked by glycosidic bonds with one or more sulfate groups. This term encompasses herein mono- and heteromeres, and linear and branched forms. It further encompasses natural, synthetic, and semisynthetic sulfated polysaccharides and any artificial modification of natural polysaccharides, including sulfation (also sulfurylation).

The terms ***"hydrophobic*/*hydrophilic polymers"*** broadly refer herein to any type charged and non-charged polymers, water soluble and water insoluble polymers, and hydrophobic polymers bound or conjugated to surfactants and other amphiphilic agents. It further encompasses natural, synthetic, and semisynthetic hydrophilic and hydrophobic polymers and artificial modifications of the natural polymers.

The term ***"copolymer"*** implies a conjoint polymer made of two or more different polymers.

The terms "***crosslinked*/*partially crosslinked"*** broadly refer herein to any type of non-covalent bonding between the sulfated polysaccharide polymers and the hydrophobic or hydrophilic polymers or copolymers.

In some embodiments the sulfated polysaccharide polymers and the hydrophobic or hydrophilic polymers or copolymers are crosslinked or partially crosslinked by an ionic bond. The extent or the degree of crosslinking, the number of groups that interconnect the two types polymers or the density of cross-link connections can vary.

A degree of crosslinking (DC) is generally expressed in terms of a proportion of crosslink groups in the composite polymer (mole percent or mole fraction). A higher DC denotes more linkages per length of the polymer (or a higher density of gel component), e.g., fluids have DC close to zero, elastomers low DC and resins high DC.

The present compositions can be in liquid, semi-liquid, semi-solid forms as gels and resins. In some embodiments the compositions can be in a solid form.

In some embodiments the sulfated polysaccharide polymers and the hydrophobic or hydrophilic polymers or copolymers can be crosslinked or partially crosslinked by one or more positively charged ions within a range of DC values of at least about 1%, 5%, 10%, 15%, 20%, 25%, 30%, 35%, 40%, 45%, 50%, 55%, 60%, 65%, 70%, 75%, 80%, 85%, 90%, 95% mole percent.

The term ***"positively charged ions"*** broadly refers herein to any ion, atom or group of atoms with one or more positive charges (also a cation). It encompasses herein mono- , di-, tri- charged ions, such as metal ions of Na⁺, K⁺, Ca²⁺, Mg²⁺, Cu²⁺, Zn²⁺ and Fe²⁺ and Fe³⁺, and multiply charged or polyatomic ions such as NH₄⁺ and H₃O⁺.

In some embodiments the sulfated polymers and the hydrophobic or hydrophilic polymers can be partially crosslinked by at least one divalent cation.

In further embodiments the divalent cation can be selected from Ba²⁺, Be²⁺, Ca²⁺, Co²⁺, Mg²⁺, Cu²⁺, Ni²⁺, Fe²⁺ and/or Zn²⁺.

In some embodiments the crosslinking cation is Ca²⁺.

One of the important features of the present compositions is in having a substantially constant viscosity at RT. The term ***"substantially constant viscosity"*** implies herein that viscosity or rheological behavior of these compositions, as measured by various methods (e.g., a viscometer, a rheometer) and under various conditions (e.g., shear forces, time period), remains relatively unchanged/stable or constant within a range of less than or up to 25% deviation, or more specifically less than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% deviation.

Viscosity can be expressed in newton-second per square meter, Pascal per second or SI units (SI (µ)=Pascal per second (Pa*s⁻¹)=1 kg m⁻¹s⁻¹).

In some embodiments the term ***"viscosity"*** refers herein to a viscosity measured by a viscometer.

In some embodiments the term ***"viscosity"*** implies herein a rheological behavior and refers to a viscosity measured under varying shear forces (also shear rates) by a rheometer.

Therefore, in some embodiments the viscosity of the present composition can be expressed in terms of a substantially constant viscosity at RT in a range between about 1 mPa*s⁻¹ and about 50 mPa*s⁻¹, as measured by a viscometer, or more specifically in a range between about 1-5 mPa*s⁻¹, 5-10 mPa*s⁻¹, 10-15 mPa*s⁻¹, 15-20 mPa*s⁻¹, 20-25 mPa*s⁻¹, 25-30 mPa*s⁻¹, 30-35 mPa*s⁻¹, 35-40 mPa*s⁻¹, 40-45 mPa*s⁻¹, 45-50 mPa*s⁻¹, or any other ranges derived from the above.

In some embodiments said viscosity can be in a range between about 1-50 mPa*s⁻¹, 5-45 mPa*s⁻¹, 10-40 mPa*s⁻¹, 15-35 mPa*s⁻¹, 20-30 mPa*s⁻¹.

In some embodiments said viscosity can be in a range between about 1-50 mPa*s⁻¹, 1-45 mPa*s⁻¹, 1-40 mPa*s⁻¹, 1-35 mPa*s⁻¹, 1-30 mPa*s⁻¹, 1-25 mPa*s⁻¹, 1-20 mPa*s⁻¹, 1-15 mPa*s⁻¹, 1-10 mPa*s⁻¹, 1-5 mPa*s⁻¹.

Viscosity is dependent on the concentrations of the polymer components (the sulfated polysaccharides and hydrophobic or hydrophilic polymers) and the crosslinkers (positively charged ions) and/or the degree of crosslinking.

One of the distinctive features of the present compositions is that the viscosity, as measured by a viscometer, remains substantially constant within the above ranges for a period of at least 1, 2, 3, 4, 5, 6, 7, 8, 9, 10, 11, 12, 13, 14, 15, 16, 17, 18, 19, 20, 21 days.

Another distinctive feature is that their viscosity, as measured by a viscometer, is lower than the viscosity of analogous solutions with the same combinations and concentrations of polymers but with no positively charged ions or no crosslinking.

In some embodiments said viscosity can be up to 20% lower than the viscosity of solutions with the same concentrations of the same polymers but with no positively charged ions when measured under the same conditions., or up to 20%, 19%, 18%, 17%, 16%, 15%, 14%, 13%, 12%, 11%, 10%, 9%, 8%, 7%, 6%, 5%, 4%, 3%, 2%, 1% lower than the viscosity of solutions with the same concentrations of the same polymers but with no positively charged ions.

In other words, crosslinking or a partial crosslinking confers to the compositions a lower viscosity or a more liquid, liquified state, which counterintuitive and surprising, and is characteristic of the compositions when presented alone, before contacting a cellular or a mucosal surface. This structural feature may have significant advantages in terms of ease of application, spread-ability and spray-ability of the present compositions, especially in the context of topical applications or applications onto oral and/or nasal mucosal.

Yet another distinctive feature of the present compositions is that upon contact with a cellular or a mucosal surface they become transformed (transformable) into a film with surprising properties of continuity, durability and intactness and the ability to provide sufficient and uniform coverage. This structural feature of a transformability into a film is characteristic of the compositions after contacting a cellular or a mucosal surface and is essentially responsible for the biological advantages of the mucoadhesiveness and mucoprotectivity of these compositions.

Thus, in some embodiments, before contact with a cellular or a mucosal surface, the compositions can be in a liquid, a semiliquid or a sprayable form.

In some embodiments, before contact with a cellular or a mucosal surface, the compositions can be in a liquid, a semiliquid or a sprayable form.

In some embodiments, upon contact with a cellular or a mucosal surface, the compositions can be transformed or are transformable into a uniform and continuous film.

In some embodiments the cellular or the mucosal surface can be an epithelial mucosa. The term ***"epithelial mucosa"*** encompasses herein oral, nasal mucosa, and further, mucosa lining of the eyelids, cornea trachea, lungs, stomach and intestines, the ureters, urethra, and urinary bladder, genitals, and the anal canal.

Thus, in some embodiments the compositions can be transformed into a uniform, and continuous film upon contact with oral and/or nasal mucosa.

The film forming ability of the present compositions is rooted in their unique rheological behavior, revealed in a substantially constant viscosity under varying shear forces, as measured by a rheometer.

In some embodiments said viscosity can be expressed in terms a substantially constant viscosity in a range between about 100 mPa*s⁻¹ and about 800 mPa*s⁻¹ for a shear rate in the range between about 0.1 s⁻¹ and about 10 s⁻¹, or more specifically in a range between about 100-200 mPa*s⁻¹, 200-300 mPa*s⁻¹, 300-400 mPa*s⁻¹, 400-500 mPa*s⁻¹, 500-600 mPa*s⁻¹, 600-700 mPa*s⁻¹, 700-800 mPa*s⁻¹ for a shear rate of up to 0.1 s⁻¹, 1 s⁻¹, or 10 s⁻¹, or any other ranges derived from the above.

In some embodiments said viscosity can be in a range between about 100-800 mPa*s⁻¹, 150-750 mPa*s⁻¹, 200-700 mPa*s⁻¹, 250-650 mPa*s⁻¹, 300-600 mPa*s⁻¹, 350-550 mPa*s⁻¹, 400-500 mPa*s⁻¹ for a shear rate of up to 0.1 s⁻¹, 1 s⁻¹, or 10 s⁻¹.

In some embodiments said viscosity can be in a range between about 100-800 mPa*s⁻¹, 100-750 mPa*s⁻¹, 100-700 mPa*s⁻¹, 100-650 mPa*s⁻¹, 100-600 mPa*s⁻¹, 100-550 mPa*s⁻¹, 100-500 mPa*s⁻¹, 100-450 mPa*s⁻¹, 100-400 mPa*s⁻¹, 100-350 mPa*s⁻¹, 100-300 mPa*s⁻¹, 100-200 mPa*s⁻¹ for a shear rate of up to 0.1 s⁻¹, 1 s⁻¹, or 10 s⁻¹.

In some embodiments, said viscosity may have less than or up to 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1% deviation for varying shear rates in the range between about 0.1 and about 10 s⁻¹, or a deviation in a range of between about 1%-25%, 1%-20%, 1%-15%, 1%-10%, 1%-5% deviation for a shear rate of up to 0.1 s⁻¹, 1 s⁻¹, or 10 s⁻¹.

This feature makes the present compositions essentially distinct from analogous non-crosslinked solutions with the same combinations and concentrations of polymers and compositions with single polymers that have a completely different rheological behavior, with significant fluctuations of viscosity under the same conditions.

As has been noted, in some embodiments the compositions of the invention can be provided as liquid and semi-liquid formulations and sprays, that can be especially useful for topical applications and application onto oral and nasal mucosa.

In some embodiments said formulation can comprise micro or nano particles or droplets. This type of compositions can be useful for applications via inhalation and assisted by inhalation devices.

In some embodiments the droplets or particles can have a size in a range of more than 1 µm or in a range between about 1-10 µm, 10-20 µm, 20-30 µm, 30-40 µm, 40-50 µm, 50-60 µm, 60-70 µm, 70-80 µm, 90-100 µm, or alternatively, in a range of less than 1 µmor in a range of between about 1-100 nm, 100-200 nm, 200-300 nm, 300-400 nm, 400-500 nm, 500-600 nm, 600-700 nm, 700-800 nm, 800-900 nm, 900-1000 nm.

In some embodiments, the droplets or particles can encapsulate various types of actives to provide added features of increased surface area, drug loading and porosity, thereby facilitating local and/or systemic delivery and adsorption of these actives.

In some embodiments the compositions can be provided in a solid or semi-solid film or a resin. This type of compositions can be useful for local or topical applications for localized or systemic drug delivery.

All these properties make the present compositions highly adaptable to various formulation technologies and drug delivery systems, either per se or in combination with other actives or drugs to achieve added therapeutic value. Non-limiting examples of such formulations are gel/spray and inhalation materials for oral and/or nasal applications, mucoadhesive films and resins for applications onto mucosal tissues, adhesive films and resins for localized topical or surgical applications.

On the subject of polymers, in numerous embodiments the sulfated polysaccharide polymers and the hydrophobic or hydrophilic polymers comprised in the compositions of the invention can be characterized as biocompatible polymers. The term ***'biocompatible'*** broadly refers to any material which is non-harmful to the living tissues.

In some embodiments the polymers can be natural polymers obtained from plants, animals, and various microorganisms, modified natural polymers or synthetic polymers, or any combination thereof. Examples of are sulfated polysaccharides obtained from algae or marine invertebrates (e.g., carrageenan, fucan), and glycosaminoglycans obtained from animals (e.g., heparin), plant polysaccharides (e.g., starch, cellulose, pectin), and polysaccharides from other sources such as bacteria (e.g., dextran) and exoskeleton of arthropods and fungi cell wall (e.g., chitin).

In some embodiments at least one sulfated polysaccharide polymer can be selected from natural or modified sulfated polysaccharides such as galactans, ulvans, fucan, fucoidans, heparins, sulfated glucosamines, sulphated chitosan, chitin and/or chondroitin and sulfated dextran, or any combination thereof.

In some embodiments at least one sulfated polysaccharide polymer can be a linear and/or non-linear sulfated polysaccharide.

In some embodiments at least one sulfated polysaccharide polymer can belong to the carrageenan family of biopolymers.

The term ***"carrageenan"*** generally refers herein to a sulphated linear polysaccharide of D-galactose and 3, 6-anhydro-D-galactose, which can be extracted from red seaweeds of the Rhodophyceae class, for example. This term encompasses all classes of carrageenan, including the three predominant classes, kappa (k), iota (i), and lambda (λ) that differ in their chemical and physical properties, methods of production and other properties, and the ability to interact with other biopolymers and film forming abilities. For example:
The ***iota carrageenan*** forms into a strong gel when combined with calcium salts. It contains 28 to 30% of ester sulfate content. It is soluble in water and is elastic in texture.

The ***kappa carrageenan,*** the most common form of carrageenan, is considered a food grade. It contains 25 to 30% of ester sulfate content. It produces a strong gel when combined with potassium salts and forms into a brittle gel when mixed with locust bean gum.

The ***lambda carrageenan*** differs from the two forms in that it is soluble in cold water, whereas the above forms are soluble in hot water. It is applied to obtain high viscous property in product.

The chemical structure of the iota, kappa and lambda carrageenan is shown below.

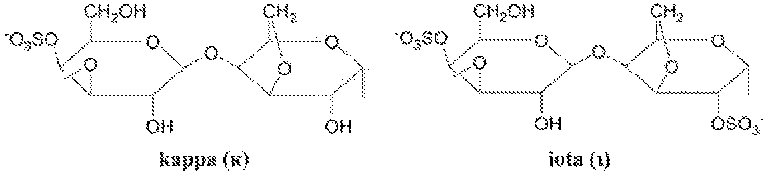

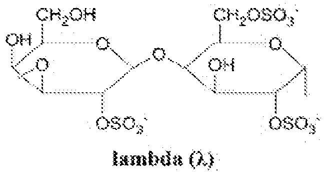

In some embodiments least one sulfated polysaccharide polymer can be iota carrageenan, kappa carrageenan and/or lambda carrageenan.

On the hydrophilic and hydrophobic polymers or co-polymers, in some embodiments the compositions can comprise at least one polymer selected from poly(glycolic acid) (PGA), poly(lactic acid) (PLA), polydioxanoes, polyoxalates, poly(α-esters), polyanhydrides, polyacetates, polycaprolactones, poly (orthoesters), polyamino acids, polyaminocarbonates, polyurethanes, polycarbonates, polyamides, poly(alkyl cyanoacrylates), mixtures and copolymers thereof.

In some embodiments the compositions can comprise at least one polymer selected from stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates, lactide/glycolide co-polymers, polyanhydrides and combinations thereof.

In some embodiments the compositions can comprise at least one polymer selected from polysaccharides, gums, and combinations thereof. Gum herein refers to a complex branched heteropolysaccharide which neutral or charged (anionic or cationic). Notable examples are Arabic and Xanthan gums.

In some embodiments the compositions can comprise natural or synthetic hydrophilic polymers selected from polyethylene oxide (PEO), pullulan, hydroxypropylmethyl cellulose (HPMC), hydroxyethyl cellulose (HPC), hydroxypropyl cellulose, polyvinyl pyrrolidone, carboxymethyl cellulose, polyvinyl alcohol, sodium alginate, polyethylene glycol, xanthan gum, tragancanth gum, guar gum, acacia gum, arabic gum, polyacrylic acid, methylmethacrylate copolymer, carboxyvinyl copolymers, starch, gelatin, ethyl cellulose, hydroxypropyl ethyl cellulose, cellulose acetate phthalate, hydroxypropyl methyl cellulose phthalate and combinations thereof.

In some embodiments at least one hydrophilic polymer can be a cationic, an anionic and/or a neutral hydrophilic polymer or a mixture thereof.

In some embodiments at least one hydrophilic polymer can be a polycation polymer or a mixture thereof. A polycation polymer refers herein to a molecule or complex having one or more positive charges at various sites.

In some embodiments the polycation polymer can be polyethyleneimine, polybrene and/or poly(amidoamine) (PAMAM).

In some embodiments the hydrophilic polymers can be polysaccharides and/or proteins.

In some embodiments at least one natural hydrophilic polymer can be a polysaccharide, or a mixture selected from dextrans, alginates, chitosans, agaroses and/or pullulans obtained from natural sources, plants, alga, animals, fungi and various microorganisms.

In some embodiments at least one natural hydrophilic polymer can be a protein selected from albumins, gelatins, collagens, lectins, legumines, and/or vicilines, or a combination thereof.

In some embodiments at least one natural hydrophilic polymer can be an alginate or an alginate salt.

The term ***"alginate"*** (also alginic acid or algin) generally refers herein to a naturally occurring anionic polymer, typically obtained from a brown seaweed. It encompasses a range of polymers having a linear structure of heteropolysaccharide composed of D-mannuronic acid and L-guluronic acid, with various degrees of viscosity. It further encompasses alginates from various sources, with different M-G content, and different physical properties. An alternative source of alginates is bacterial biosynthesis (*Azotobacter* and *Pseudomonas*), which are different from seaweed-derived alginates by chemical structure and physical properties.

The chemical structure of a typical alginate is shown below.

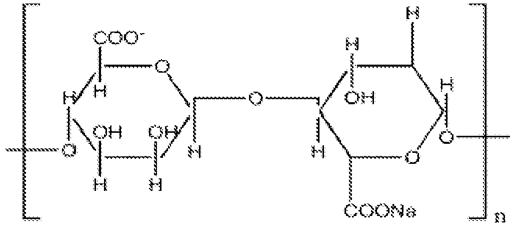

This term further encompasses various alginate salts with metals such as sodium, potassium, calcium, and magnesium alginate salts. The salts can contain various types of alginates or mixtures thereof. The molecular weight of commercially available sodium alginates, for example, ranges between 32,000 and 400,000 g/mol.

In general, the compositions of the invention can comprise various combinations of sulfated polysaccharide polymers, hydrophobic or hydrophilic polymers and crosslinking positively charged ions, and more than one agent from each of these groups.

In some embodiments the compositions can comprise at least one carrageenan and at least one alginate crosslinked or partially crosslinked by Ca²⁺.

In some embodiments the carrageenan can be at a concentration in a range between about 0.005%-1%, or in a range between about 0.005%-0.01%, 0.01%-0.05%, 0.05%-0.1%, 0.1%-0.2%, 0.2%-0.3%, 0.3%-0.4%, 0.4%-0.5%, 0.5%-0.6%, 0.6%-0.7%, 0.7%-0.8%, 0.8%-0.9%, 0.9%-1% (w/w).

In some embodiments the alginate can be at a concentration in a range between about 0.1%-3% (w/w), or in a range between about 0.1%-0.5%, 0.5%-1%, 1%-1.5%, 1.5%-2%, 2%-2.5%, 2.5-3% (w/w).

In some embodiments the Ca²⁺ can be at a concentration in a range between about 0.0001%-1% (w/w), or in a range between about 0.0001%-0.0005%, 0.0005%-0.001%, 0.001%-0.005%, 0.005%-0.01%, 0.01%-0.05%, 0.05%-0.1%, 0.1%-0.5%, 0.5%-1% (w/w).

In some embodiments the carrageenan can be at a concentration in a range of between about 0.1%-0.3% (w/w), or at a concentration of up to about 0.1%, 0.125%, 0.15%, 1.75%, 0.2%, 0.225%, 0.25%, 0.275%, 0.3% (w/w).

In some embodiments the alginate can be at a concentration in a range of between about 1%-3% (w/w), or at a concentration of up to about 1%, 1.25%, 1.5%, 1.75%, 2%. 2.25%, 2.5%, 2.75%, 3% (w/w).

In some embodiments the Ca²⁺ can be at a concentration in a range of between about 0.0001%-0.005% (w/w), at a concentration of up to about 0.0001%, 0.0005%, 0.001%, 0.0015%, 0.002%, 0.0025%, 0.003%, 0.0035%, 0.004%, 0.0045%, 0.005% (w/w).

In some embodiments the carrageenan can be at a concentration in a range of between about 0.2%-0.3% (w/w), or at a concentration of up to about 0.2%, 0.225%, 0.25%, 0.275%, 0.3% (w/w).

In some embodiments the alginate can be at a concentration in a range of between about 1%-3% (w/w), or at a concentration of up to about 1%, 1.25%, 1.5%, 1.75%, 2%. 2.25%, 2.5%, 2.75%, 3% (w/w).

In some embodiments the Ca²⁺ can be at a concentration in range of between about 0.01%-0.03% (w/w), or at a concentration of up to about 0.01%, 0.0125%, 0.015%, 0.0175%, 0.02%, 0.0225%, 0.025%, 0.0275%, 0.03% (w/w).

This type of compositions can be appliable to oral and/or nasal spray/gels and inhalation products.

Still in other embodiments the compositions can comprise higher concentrations of Ca²⁺ such as Ca²⁺at a concentration in a range of between about 1%-2% (w/w), or up to about 1%, 1.1%, 1.2%, 1.3%, 1.4%, 1.5%, 1.6%, 1.7%, 1.8%, 1.9%, 2% (w/w).

This type of compositions can be appliable to solid forms and inhalation products.

The present compositions can further comprise various solvents, buffers, excipients, adjuvants, permeation enhancers, plasticizers, thickeners, rheological modifiers, alcohols, extenders, preservatives, colorants, pigments, sweeteners, flavoring and/or odor agents, or combinations thereof.

In some embodiments the solvents can be selected from water, alcohol, acetone, methylene chloride and combinations thereof.

The present compositions can be used as a basis for pharmaceutical compositions with actives provided in therapeutically effective doses, and pharmaceutically acceptable buffers and/or excipients.

In some embodiments the sulfated polysaccharide polymers and the hydrophobic or hydrophilic polymers can serve as actives when provided in therapeutically effective doses.

The terms ***"therapeutically effective amount** "* or ***"dose"*** (also pharmacologically, pharmaceutically, or physiologically effective amount or dose) broadly relate to the amounts or doses of a composition or an active required to provide the desired level of physiological or clinically measurable response. The term dose can further relate to a number of dosage forms or number of administrations to produce the desired effect. The effect can be assessed or measured by specific clinical indices of the relevant condition, recognized molecular or biochemical markers and other parameters. In this case, these terms can be translated to an amount or dose of the pharmaceutical composition that can be related to a measurable mucoprotective effect, and specifically, a measurable reduction of infection or viral load in the mucosal tissue, or a reduction of inflammatory response by one or more recognized inflammatory markers.

In some embodiments the pharmaceutical compositions can further comprise at least one additional active or therapeutic agent. In terms of added therapeutic actives, the present pharmaceutical compositions can incorporate various types of drugs and therapeutic actives, depending on the indication, application method and use.

In numerous embodiments the pharmaceutical compositions can incorporate therapeutic agents conferring protection an oral and nasal mucosal barrier function. The concept of ***"mucosal barrier function**"* has been discussed above. Essentially, it encompasses any dysregulation of homeostasis of oral and/or nasal epithelial or mucosal barrier, including physical disruption of the epithelial tissue and/or specific molecular structures such as TJs, dysregulated secretion of pro-inflammatory cytokines, growth factors and chemokines responsible for innate and adaptive immunity, insufficient secretion of antimicrobial peptides and dysregulation of mucociliary clearance by ciliated epithelial cells responsible for trapping of microbes and microparticles.

It can be presumed that due to their mucoadhesive and mucoprotective properties and their ability to prevent or mitigate an excessive exposure of the mucosa to harmful and infectious pathogens and allergens, the present compositions can contribute to the restoration of the oral and/or nasal mucosal barrier and its functionality.

In some embodiments the pharmaceutical compositions can be applied for treating, alleviating and/or preventing microbial oral and/or nasal infections, which encompasses herein bacterial and viral infections.

The most common examples are bacterial infections responsible for dental cavities, predominantly infections caused by *Streptococcus mutans* and *Streptococcus sobrinus,* and lactobacilli. Another example is nasal acute bacterial rhinosinusitis (ABRS) in the nasal cavity and sinuses caused by *Streptococcus pneumonia.* ABRS is usually secondary to a viral infection. Still another example are acute upper respiratory infections (URI), i.e., sinusitis, epiglottitis, laryngitis, and bronchitis, caused by group A or group C beta-hemolytic streptococci, *Corynebacterium diphtheriae* (diphtheria), *Neisseria gonorrhoeae* (gonorrhea) and *Chlamydia pneumoniae* (chlamydia).

In some embodiments the compositions can be applied for treating, alleviating and/or preventing meningococcal meningitis, a bacterial meningitis caused by *Streptococcus pneumoniae* typically found in the respiratory tract, sinuses, and nasal cavity. Indicators of this condition is sudden high fever, severe headache, seizures, stiff neck, nausea, sensitivity to light, among others.

In some embodiments, they can comprise agents targeting bacterial infections, or in other words, they can comprise various types of antibiotics and combinations of antibiotics, with various range antibacterial activities. Non limiting examples are:
1. Penicillins such as penicillin and amoxicillin
2. Cephalosporins such as cephalexin (Keflex)
3. Macrolides such as erythromycin (E-Mycin), clarithromycin (Biaxin), and azithromycin (Zithromax)
4. Fluoroquinolones such as ciprofolxacin (Cipro), levofloxacin (Levaquin), and ofloxacin (Floxin)
5. Sulfonamides such as co-trimoxazole (Bactrim) and trimethoprim (Proloprim)
6. Tetracyclines such as tetracycline (Sumycin, Panmycin) and doxycycline (Vibramycin)
7. Aminoglycosides such as gentamicin (Garamycin) and tobramycin (Tobrex)

Sinusitis is a specific example as it can be caused by viruses, bacteria, and/or fungi and also aeroallergens.

In some embodiments the compositions can be applied for treating, alleviating and/or preventing infections caused by human respiratory viruses. This group includes a broad range of viruses having a common propensity to replicate in cells of the respiratory tract, and that are usually spread by respiratory discharges to other hosts. The term ***"respiratory viruses"*** encompasses herein various types of influenza viruses, respiratory syncytial virus (RSV), parainfluenza viruses, metapneumoviruses, rhinoviruses, coronaviruses, adenoviruses, and bocaviruses.

In some embodiments the compositions can be applied for treating, alleviating and/or preventing viral infections in the oral and/or nasal cavities.

In some embodiments the compositions can be applied in combination with known antiviral drug therapies. A number of antiviral drugs currently available, including amantadine (Symmetral), ribavirin (Virazole) and palivizumab (Synagis), the two latter are indicated for RSV. Oral amantadine is effective for both treatment and prevention of influenza A. Additional drugs are in various stages of R&D and regulatory approval.

In some embodiments the compositions can be used for treating, alleviating and/or preventing a common cold, a mild flu, tonsillitis, laryngitis, and sinus infection. Currently, the four FDA-approved drugs for the treatment of flu are oseltamivir phosphate (Tamiflu), zanamivir (Relenza), peramivir (Rapivab) and baloxavir marboxil (Xofluza).

In some embodiments the compositions can be applied for treating, alleviating and/or preventing infections of influenza virus A and B, the main causative agents of seasonal epidemics of influenza (the seasonal flu), alone or in combination with Arbidol (an FDA-approved drug against influenza).

In some embodiments the compositions can be applied for treating, alleviating and/or preventing aseptic viral meningitis predominantly caused by enteroviruses.

More broadly, the compositions can be applied for treating, alleviating and/or preventing any type of infections caused, related or associated with influenza viruses, respiratory syncytial virus, parainfluenza viruses, metapneumovirus, rhinovirus, coronaviruses, adenoviruses, and bocaviruses.

In some embodiments the compositions can be applied for treating, alleviating and/or preventing infections caused by SARS-CoV-2, including known and more recent SARS-CoV-2 variants.

In some embodiments the compositions can be applied together with additional antiviral drugs interfering with viral entry and life cycle (a broad-spectrum antiviral activity), such as nucleoside/nucleotide analogs (e.g., Remdesivir), protease inhibitors (disulfiram, lopinavir, and ritonavir and darunavir) and multiple small molecules designed on the basis specific viral structures.

In some embodiments the compositions can comprise antiviral drugs targeting specific viruses.

In some embodiments the compositions can further comprise at least antibiotic such as Penicillin VK, Amoxicillin, Penicillin G benzathine, Cefadroxil, Erythromycin, the wide-range Augmentin (Amoxicillin and clavulanic acid).

In some embodiments the compositions can comprise a combination of antibiotics and antiviral drugs for treating, alleviating and/or preventing primary and secondary infections.

In some embodiments the compositions can comprise antiviral vaccines containing either an inactivated or attenuated virus, or parts of a viral genome. A precedent COVID-19 vaccine in a form of nasal spray has been recently tested in animals.

In some embodiments the compositions can be applied for treating and/or preventing aeroallergens induced allergy and/or inflammation. Aeroallergens broadly refers herein to a wide range of airborne substances or inhalants, such as pollens, spores, and other biological or non-biological airborne particles that can cause allergic reaction.

In clinical terms, in some embodiments the compositions can be applied for treating, alleviating and/or preventing cough, wheeze, and episodic respiratory distress resulting from aeroallergens driven Th2 immune response.

In some embodiments the compositions can be applied for treating, alleviating active asthma and/or preventing a risk of developing asthma.

In some embodiments the compositions can be applied for preventing occupational hazards. Occupational allergens involve hundreds of chemicals present in virtually every industry (e.g., metals, epoxy and acrylic resins, rubber additives, and chemical intermediates).

In some embodiments the compositions can further comprise steroidal and non-steroidal anti-inflammatory drugs. Examples are various types of corticosteroids (cortisone, hydrocortisone, prednisone, prednisolone, dexamethasone), and various types of NSAIDs such as Aspirin, Ibuprofen, Naproxen, Meloxicam Celecoxib, and others.

In some embodiments the compositions can further comprise antiallergic drugs. Notable examples are antihistamines. Nasal sprays can include decongestant such as Oxymetazoline, Tetrahydrozoline, and various types of corticosteroids (Budesonide, Fluticasone furoate, Fluticasone propionate, Mometasone, Triamcinolone). Inhalers can include corticosteroids with long-acting bronchodilators such as Beclomethasone, Budesonide, Ciclesonide, Fluticasone, Mometasone, and additional agents targeting specific disorders of mucosal barrier function.

Broadly speaking, the present compositions can incorporated into of several types of products:
(1) OTC-type of products with the compositions per se or in conjunction with OTC approved or homeopathic actives for protecting the nasal and oral mucosa against exposure to potential infectious and non-infection agents and pathogens.
(2) Prescription based products with the compositions and FDA approved drugs targeting clinical conditions related to oral and nasal mucosal barrier dysfunction.
(3) Drug delivery systems with the compositions serving as carriers for FDA approved drugs for various clinical disorders and clinical and subclinical conditions.

For example, in some embodiments the invention can provide oral and nasal sprays/gels comprising one or a combination of the above compositions.

In some embodiments the invention can provide oral or nasal inhalation materials comprising one or a combination of the above compositions. The inhalation material can be in the form of particles or droplets, preferably larger than 10 µm.

In some embodiments the spray/gel or the inhalation materials can serve for protecting the oral and/or nasal mucosa against disorders of mucosal barrier function.

In some embodiments the spray/gels or the inhalation materials can further comprise at least one therapeutic agent targeting disorders of mucosal barrier function.

In yet another aspect the invention can provide kits comprising one or a combination of compositions of the invention (e.g., an oral or a nasal spray/gel or an inhalation material) and a device for oral or nasal delivery, and instructions for use. The concept of kit implies compartmentalization of the spray/gel or the inhalation material into specific containers or dosage forms.

In some embodiments the compositions can be incorporated into the device.

In another aspect the invention can provide an oral or a nasal drug delivery system comprising one or a combination of the above compositions, with or without additional therapeutic agents.

It is yet another important objective of the invention to provide drug delivery systems targeting various clinical disorders and clinical and subclinical conditions. Drugs or agents delivered by such systems are usually referred to as systemically acting agents or drugs. The term *'**systemically acting agents or drugs**'* broadly refers herein to drugs acting beyond the point of their application or administration and targeting other part of the body via circulation. This term encompasses herein drugs acting on cardiovascular, respiratory, gastrointestinal or nervous systems, and psychiatric drugs. On the molecular level, it encompasses herein enzyme inhibitors, receptor antagonists or agonists, proton-pump and ion-channel inhibitors, and reuptake inhibitors.

In some embodiments additional systemic drugs can be selected from antibiotics, antifungal agents, antiviral agents, neuroleptics, analgesics, hormones, anti-inflammatory drugs, non-steroidal anti-inflammatory drugs, anti-rheumatic, drugs anticoagulants, beta-blockers, diuretics, anti-hypertension drugs, anti-atherosclerosis drugs, antidiabetics, anti-asthmatic drugs, decongestant and cold medicines.

Candidate agents can be selected from the general list of FDA-recognized drug categories, an example of which is provided in **ANNEX A.** A nonlimiting list of relevant clinical disorders and conditions is provided in **ANNEX B.**

In some embodiments additional therapeutic agents can be selected from beneficial oils, nutraceuticals, vitamins, dietary or food supplements, nutrients, antioxidants, superfoods, natural extracts of animal or plant origin, probiotic microorganisms, or any combination thereof.

In structural terms, the compositions can comprise therapeutic agents belonging to the groups of carbohydrates, lipids, proteins, peptides, enzymes, nucleic acids, oligonucleotides or composite molecules comprising thereof.

In some embodiments the compositions can comprise agents belonging to the groups of small molecules. A small molecule usually refers a low molecular weight (<900 Daltons) organic compound that may regulate a biological process.

In some embodiments the compositions can comprise single-stranded and double stranded DNA or RNA, siRNA, plasmids, linear or circular DNA or RNA, or combinations thereof.

It is another objective of the invention to provide methods for systemic or local drug delivery via epidermal mucosa by administering to the epidermal mucosa of a subject one or more of the above compositions or delivery systems. The term ***"administering to epidermal mucosa"*** implies herein oral, nasal mucosa, ocular mucosa, lung mucosa, and other mucosa tissues.

In some embodiments the methods of the invention can involve concurrent administering to the subject of one or more therapeutic agents, as per specific indication and subject's needs, said administering can be simultaneous or successive employing enteral, parenteral, topical or any other route.

In some embodiments the methods can be applied for protecting an oral and nasal mucosal barrier function in a subject by oral or nasal administering one or a combination of the above compositions.

In some embodiments the methods can be applied for treating, alleviating, and preventing microbial oral and/or nasal infections in a subject by oral or nasal administering a therapeutically effective amount of any of the above compositions.

In some embodiments the methods can be applied for treating, alleviating, and preventing an aeroallergen induced allergy and/or inflammation in a subject by oral and/or nasal administering a therapeutically effective amount of any of the above compositions.

The invention can be further articulated in a form of use of any of the above compositions for the manufacture of a medicament for treating an aeroallergen induced allergy and/or inflammation or a microbial oral or nasal infection.

From yet another point of view, the mucoadhesive compositions of the invention can be employed in the bioengineering of functional tissue implants. Owing to their biocompatibility and physical, chemical, and biological properties, the present compositions can serve as a universal "fits all" material in the form of a matrix or an injectable hydrogel that can be used to provide physical support for the regeneration of tissues and more complex organs and to support cell assembly to specific sites. Adaptability of the present compositions to incorporate additional materials, actives and drugs makes them particularly attractive candidates for this type of applications.

In some embodiments the matrixes made of the compositions of the invention can further comprise actives, drugs, growth factors, hormones enzymes, nucleic acids, oligoes and other agents to provide biochemical and topographical cues for cellular assembly and function.

In some embodiments such matrixes can further comprise encapsulated cells of various types and origins, depending on the required indication. Specific examples are stem cells of various origins, which upon appropriate triggering can lead to assembly, development, and differentiation of specific cells at the application site.

In other words, the present compositions can be used to assist in structural and functional restoration of damaged tissues by providing mechanical support and navigating cell fate in the context of neuronal or cardiac regeneration to repair infarcted heart, injured spinal cord, neurodegenerative brain.

When viewed as tools for neurorepair or regeneration, the present compositions can be further provided with assisting devices and incorporated into neurological surgical procedures.

The term "about" in all its appearances in the text denotes up to a ±10% deviation from the specified values and/or ranges, more specifically, up to ±1%, ±2%, ±3%, ±4%, ±5%, ±6%, ±7%, ±8%, ±9% or ± 10% deviation therefrom.

### EXAMPLES

Any method and material similar or equivalent to those described herein can be used in the practice or testing of the present invention. Some embodiments of the invention will be now described by way of examples with reference to respective figures.

### Materials and Methods

### Cells

MRC-5 human normal lung fibroblast (ATCC CCL-171) were cultured in EMEM medium with 10% FBS, 2 mM L-Glutamine, 1% Penicillin-Streptomycin (Biological Industries).

MDCK dog normal kidney cells (ATCC NBL-2) were cultured in EMEM medium with 1% FBS, 2mM L-Glutamine, 1% Penicillin-Streptomycin (Biological Industries).

A549 adenocarcinoma human alveolar basal epithelial cells (ATCC CCL-185) were cultured in EMEM medium with 10% FBS, 2 mM L-Glutamine, 1% Penicillin-Streptomycin (Biological Industries).

Vero E6 African green monkey kidney cells (ATCC CRL-1586) were cultured in EMEM medium with 5% FBS, 2 mM L-Glutamine, 1% Penicillin-Streptomycin (Biological Industries).

### Viruses

Human corona virus 229E was diluted 1:15 in the respective culture medium and introduced into cultured MRC-5 cells (infective dose 2 µL/well).

Influenza virus H1N1 was diluted 1:10 in the respective culture medium and introduced into MDCK cells (infective dose 3 µL/well).

SARS-CoC-2 Omicron variant (lineage B.1.1.529.BA.1) was diluted 1:10 in the culture medium and introduced into Vero E6 cells (infective dose 30 µL/well).

### Allergen

Recombinant Dermatophagoides pteronyssinus dust mite allergen Der p1 (a.a. 20-320 containing 6-His C-terminal tag, with total Mw=34.5kDa, pI=5.6) was diluted in the assay medium and introduced into A549 cells (final concentration 50 µg/ml).

Recombinant Dermatophagoides farina Der f1 (a.a. 1-320 fused to a 6 His Tag at C-terminus, with total Mw=36kDa, pI=5.88) was diluted in the assay medium and introduced into A549 cells (final concentration 25 µg/ml).

Recombinant Gramineous group Pollen allergen Phl p1 (a glycosylated polypeptide chain produced in SF9, calculated molecular mass=29kDa, expressed with a 10xHis tag at N-terminus and purified by a chromatographic method) was diluted in the assay medium and introduced into A549 cells (final concentration 25 µg/ml).

Recombinant Carpinus betulus Pollen allergen Car b1 isoform (a non-glycosylated polypeptide chain produced in E.coli, with calculated molecular mass=18kDa, expressed with a 10xHis tag at N-terminus and purified by a chromatographic method) was diluted in the assay medium and introduced into A549 cells (final concentrations 15 µg/ml, 30 µg/ml and 60 µg/ml).

### Pre-treatment and treatment procedures

1. Cells were plated on 96 well plates in the culture medium (1x10⁴ cells/well) and allowed to attach for 16-24 h at 37⁰C, 5% CO₂.
2. 30 µL Test Items (listed in **Table 1)** were added to the cells and incubated for 30 min at 37⁰C, 5% CO₂.

**Table 1. List of Test Items (alginate/carrageenan/CaCl₂ % w/w)**

| |
|---|
| Iota-carrageenan (0.12% or 0.24%) |
| Lambda-carrageenan (0.12% or 0.24%) |
| Alginate (2%) |
| Non-crosslinked composition 1 (2%/0.12%/0%) |
| Non-crosslinked composition 2 (2%/0.24%/0%) |
| Non-crosslinked composition 3 (2%/0%/0%) |
| Partially crosslinked composition 1 (2%/0.12%/0.02%) |
| Partially crosslinked composition 2 (2%/0.24%/0.02%) |
| Partially crosslinked composition 3 (2%/0%/0.02%) |
| Microspheres composition 1 (2%/0.12%/1.47%) |
| Microspheres composition 2 (2%/0.24%/1.47%) |
| Microspheres composition 3 (2%/0%/1.47%) |

3. Cells were pre-incubated with 60-140 µL assay medium and 30 µL tested virus or 10 µL activated allergen in the presence of 5 mM L-Cysteine for 15 min on ice.
4. Cells were incubated with the tested virus or allergen for 72 h at 37⁰C, 5% CO₂ for 72 h, washed and incubated for additional 72 h at the same conditions.
5. At the end of incubation period, cells were treated as per respective assay protocol.

### XTT-based cell proliferation assay

1. The reaction solution was prepared from 0.1 mL activation solution and 5 mL XTT reagent (Cell Proliferation Kit, Biological Industries) as per manufacturer's protocol.
2. Cells were washed and 50 µL of XTT reagent was added to the fresh medium.
3. OD was measured at 450 nm, normalizing the results to the vehicle treated cells (OD=0.5-1.5) and subtracting the non-specific OD at 620 nm (% cells viability).

### IL-8 detection by ELISA assay

1. At the end of incubation period, assay media was collected and stored (-20° C) for IL-8 ELISA assays.
2. The supernatant was tested in aliquots of 25 µL as per manufacturer's instructions.

### EXAMPLE 1: Cytotoxicity studies in several animal cell lines

Cytotoxicity of the compositions of the invention was studied in various in vitro cell models (MRC-5, MDCK, A549 and Vero cells). Cells were treated with compositions comprising various proportions of alginate/carrageenan/CaCl₂ **(Table 2),** corresponding to various degrees of crosslinking (#26 partial, #21 complete, #16 no crosslinking), and comparing to the cell viability of untreated cells (UT).

**Table 2. Compositions tested in MRC-5, MDCK, A549 and Vero cells**

| **Composition** | **Sodium alginate (%)** | **Lambda carrageenan (%)** | **CaCl₂ (%)** | **Conc. tested composition** |
|---|---|---|---|---|
| **#16** | 2 | 0.24 | 0 | 100% |
| **#21** | 2 | 0.24 | 1.47 | 100% |
| **#26** | 2 | 0.24 | 0.02 | 100% |

The toxicity results are shown in **Figs 1A-1D** (means ±SE in each group). Overall, in all the tested types of cells, the compositions demonstrated no or only minimal cytotoxicity (<5% reduction in % cell viability), with no significant effect of the degree of CaCl₂ crosslinking.

### EXAMPLE 2: Protective effect of the compositions against corona viruses

Mucoprotective effect of the compositions of the invention against spread and infection of various strains of corona viruses (229E and Omicron lineage B.1.1.529.BA.1) was studied in in vitro cell models (MRC-5 and Vero cells, respectively). In the 229E model, virus infected cells were treated with compositions comprising various concentrations of alginate/carrageenan/CaCl₂ (#25, #26, #27 partial and #4, #5, #15, #16 no crosslinking) and various concentrations of carrageenan (#16, #26 high, #4, #15, #25 low and #5, #27 no carrageenan) (see **Table 3).** In the Omicron model, virus infected cells were treated with serial dilutions (0.2 to 1) of the partially crosslinked and non-crosslinked compositions (#26 and #16, respectively). Treated cells were compared to uninfected and untreated cells (UT) and virus infected cells (UT+virus).

**Table 3. Compositions with combinations of polymers**

| **Composition** | **Sodium alginate (%)** | **Lambda carrageenan (%)** | **CaCl₂ (%)** |
|---|---|---|---|
| **#4** | 0 | 0.12 | 0 |
| **#5** | 2 | 0 | 0 |
| **#15** | 2 | 0.12 | 0 |
| **#16** | 2 | 0.24 | 0 |
| **#25** | 2 | 0.12 | 0.02 |
| **#26** | 2 | 0.24 | 0.02 |
| **#27** | 2 | 0 | 0.02 |

The results are shown in **Figs 2A-2B** and **Fig. 3** (means ±SE in each group), for the 229E and Omicron models respectively, with corresponding statistical analysis of the study groups (Tukey HSD) in **Tables 4-5,** respectively.

**Table 4. Statistical analysis of 229E study groups**

| | **UT** | **UT+V** | **#4** | **#5** | **#15** | **#16** | **#25** | **#26** | **#27** |
|---|---|---|---|---|---|---|---|---|---|
| **UT** | | p<0.01 | p<0.01 | p<0.05 | p<0.01 | p<0.05 | NS | NS | p<0.05 |
| **UT+V** | p<0.01 | | NS | p<0.01 | p<0.01 | p<0.01 | p<0.01 | p<0.01 | p<0.01 |
| **#4** | p<0.01 | NS | | NS | NS | NS | p<0.01 | p<0.01 | p<0.05 |
| **#5** | p<0.05 | p<0.01 | NS | | NS | NS | p<0.01 | p<0.01 | NS |
| **#15** | p<0.01 | p<0.01 | NS | NS | | NS | p<0.01 | p<0.01 | NS |
| **#16** | p<0.05 | p<0.01 | NS | NS | NS | | p<0.01 | p<0.01 | NS |
| **#25** | NS | p<0.01 | p<0.01 | p<0.01 | p<0.01 | p<0.01 | | NS | p<0.01 |
| **#26** | NS | p<0.01 | p<0.01 | p<0.01 | p<0.01 | p<0.01 | NS | | p<0.01 |
| **#27** | p<0.05 | p<0.01 | p<0.05 | NS | NS | NS | p<0.01 | p<0.01 | |

| | | | | | | | | | |
|---|---|---|---|---|---|---|---|---|---|
| NS non-significant | | | | | | | | | |

**Table 5. Statistical analysis of Omicron study groups**

| | **UT** | **UT+V** | **#16x1** | **#16x0.6** | **#16x0.4** | **#16x0.2** | **#26x1** | **#26x0.6** | **#26x0.4** | **#26x0.2** |
|---|---|---|---|---|---|---|---|---|---|---|
| **UT** | | p<0.01 | NS | p<0.05 | p<0.01 | p<0.01 | p<0.05 | p<0.01 | NS | NS |
| **UT+V** | p<0.01 | | p<0.01 | p<0.01 | p<0.01 | p<0.01 | p<0.01 | p<0.01 | p<0.01 | p<0.01 |
| **#16x1** | NS | p<0.01 | | NS | NS | p<0.01 | NS | NS | NS | NS |
| **#16x0.6** | p<0.05 | p<0.01 | NS | | NS | p<0.01 | NS | NS | NS | NS |
| **#16x0.4** | p<0.01 | p<0.01 | NS | NS | | p<0.05 | NS | NS | NS | NS |
| **#16x0.2** | p<0.01 | p<0.01 | p<0.01 | p<0.01 | p<0.05 | | p<0.01 | p<0.05 | p<0.01 | p<0.01 |
| **#26x1** | p<0.05 | p<0.01 | NS | NS | NS | p<0.01 | | NS | NS | NS |
| **#26x0.6** | p<0.01 | p<0.01 | NS | NS | NS | p<0.05 | NS | | NS | NS |
| **#26x0.4** | NS | p<0.01 | NS | NS | NS | p<0.01 | NS | NS | | NS |
| **#26x0.2** | NS | p<0.01 | NS | NS | NS | p<0.01 | NS | NS | NS | |

| | | | | | | | | | | |
|---|---|---|---|---|---|---|---|---|---|---|
| NS non-significant | | | | | | | | | | |

For the 229E model, the compositions with the partially crosslinked alginate-carrageenan combinations (#25, #26) had more significant antiviral mucoprotective effects than compositions with the non-crosslinked polymers (#4, #15, #16) or compositions with no carrageenan (#5, #27). The compositions with the lower and higher carrageenan concentrations (#25 vs. #26) had similar effects.

For the SARS-CoV-2 Omicron model, the effect of the composition with the partially crosslinked polymers was not concentration-dependent and remained significant with the lower concentration of the tested composition (#26x0.2 vs. #26x1). Moreover, even with the lower concentration, the partially crosslinked composition was more effective than the composition with non-crosslinked polymers (#26x0.2# vs.16x0.2).

Overall, it was shown that the compositions with the partially crosslinked alginate-carrageenan polymers have significant antiviral mucoprotective effects against several strains of corona viruses, including the currently prevalent Omicron variant.

### EXAMPLE 3: Protective effect of the compositions against influenza virus

Antiviral mucoprotective effect of the compositions was further tested for the human influenza virus (H1N1) in in vitro cell model. MDCK cells were treated with the same compositions **(Table 3).** Cell viability was evaluated as above. The results are shown in **Figs 4A-4B** (means ±SE in each group), with corresponding statistical analysis of the study groups (Tukey HSD) in **Table 6.**

**Table 6. Statistical analysis of H1N1 study groups**

| | **UT** | **UT+V** | **#4** | **#5** | **#15** | **#16** | **#25** | **#26** | **#27** |
|---|---|---|---|---|---|---|---|---|---|
| **UT** | | p<0.01 | p<0.01 | p<0.01 | NS | NS | NS | NS | NS |
| **UT+V** | p<0.01 | | p<0.01 | p<0.01 | p<0.01 | p<0.01 | p<0.01 | p<0.01 | p<0.01 |
| **#4** | p<0.01 | NS | | p<0.01 | p<0.01 | p<0.01 | p<0.01 | p<0.01 | p<0.01 |
| **#5** | p<0.01 | p<0.01 | p<0.01 | | p<0.01 | p<0.01 | p<0.01 | p<0.01 | p<0.01 |
| **#15** | NS | p<0.01 | p<0.01 | p<0.01 | | NS | p<0.05 | NS | NS |
| **#16** | NS | p<0.01 | p<0.01 | p<0.01 | NS | | p<0.05 | NS | NS |
| **#25** | NS | p<0.01 | p<0.01 | p<0.01 | p<0.05 | p<0.05 | | NS | p<0.01 |
| **#26** | NS | p<0.01 | p<0.01 | p<0.01 | NS | NS | NS | | p<0.05 |
| **#27** | NS | p<0.01 | p<0.01 | p<0.01 | NS | NS | p<0.01 | p<0.05 | |

| | | | | | | | | | |
|---|---|---|---|---|---|---|---|---|---|
| NS non-significant | | | | | | | | | |

The results show that the compositions with combinations of partially crosslinked polymers (#25, #26) had a more significant antiviral mucoprotective effect against the H1N1 virus compared to the compositions with non-crosslinked or single polymers (#4, #5, #15, #16, #27). More generally, the results suggest that the antiviral mucoprotective effect of these compositions is not restricted to the corona viruses and that they can be protective in the same way against influenza, and potentially other respiratory viruses.

The antiviral mucoadhesive effect of the compositions was further tested with regard to the degree of crosslinking. To that end, H1N1 infected MDCK cells were treated with the compositions comprising alginate-carrageenan combinations (#26) and various concentrations of CaCl₂ (0.0001 to 0.02%), corresponding to various degrees of crosslinking. The results are shown in **Fig. 5** (means ±SE in each group), with corresponding statistical analysis (Tukey HSD) in **Table 7.**

**Table 7. Statistical analysis of the CaCl₂ study groups**

| | **UT** | **UT+V** | **#26** | **#26** | **#26** | **#26** | **#26** | **#16** |
|---|---|---|---|---|---|---|---|---|
| | | | **0.02% CaCl₂** | **0.01% CaCl₂** | **0.005% CaCl₂** | **0.001% CaCl₂** | **0001% CaCl₂** | |
| **UT** | | p<0.01 | NS | NS | NS | p<0.01 | p<0.01 | p<0.01 |
| **UT+V** | p<0.01 | | p<0.01 | p<0.01 | p<0.01 | p<0.01 | p<0.01 | NS |
| **#26 0.02% CaCl₂** | NS | p<0.01 | | NS | NS | p<0.01 | p<0.01 | p<0.01 |
| **#26 0.01% CaCl₂** | NS | p<0.01 | NS | | NS | p<0.01 | p<0.01 | p<0.01 |
| **#26 0.005% CaCl₂** | NS | p<0.01 | NS | NS | | p<0.01 | p<0.01 | p<0.01 |
| **#26 0.001% CaCl₂** | p<0.01 | p<0.01 | p<0.01 | p<0.01 | p<0.01 | | p<0.01 | p<0.01 |
| **#26 0.0001% CaCl₂** | p<0.01 | p<0.01 | p<0.01 | p<0.01 | p<0.01 | p<0.01 | | p<0.05 |
| **#16** | p<0.01 | NS | p<0.01 | p<0.01 | p<0.01 | p<0.01 | p<0.05 | |

| | | | | | | | | |
|---|---|---|---|---|---|---|---|---|
| NS non-significant | | | | | | | | |

The results show that the compositions with the higher CaCl₂ concentrations (#26 0.005%-0.02% CaCl₂) had a more significant antiviral mucoprotective effect compared to the compositions with lower CaCl₂ concentrations (#26 0.001%-0.0001% CaCl₂) or no CaCl₂ (#16). Overall, the results suggest that the degree of partial crosslinking of the two polymers contributes to the desired antiviral mucoadhesive effect.

### EXAMPLE 4: Protective effect of the compositions against allergens

Mucoprotective effect of the compositions was further studied with regard to the presence of common allergens and allergen induced inflammation, using respective in vitro cell models (house dust mite allergens Der p1 and Der f1 and pollen allergens Phl p1 and Car b1) and Interleukin 8 (IL-8) as a molecular marker of inflammation. The tested compositions are listed in **Table 8.**

**Table 8. Compositions tested in the model of allergic inflammation**

| **Composition** | **Sodium alginate (%)** | **Carrageenan (%)** | **CaCl₂ (%)** |
|---|---|---|---|
| **#3** | 0 | Iota 0.12 | |
| **#4** | 0 | Lambda 0.12 | 0 |
| **#5** | 2 | 0 | 0 |
| **#15** | 2 | Lambda 0.12 | 0 |
| **#16** | 2 | Lambda 0.24 | 0 |
| **#25** | 2 | Lambda 0.12 | 0.02 |
| **#26** | 2 | Lambda 0.24 | 0.02 |

A549 cells were treated with the compositions comprising various concentrations of alginate/carrageenan/CaCl₂ (#25, #26 partial and #4, #5, #15, #16 no crosslinking) and various concentrations of Lambda (#16, #26 high, #4, #15, #25 low and #5 no carrageenan) or Iota carrageenan (#3), using two concentrations of the tested compositions (x1 or x0.5) and comparing treated and untreated (UT) cells and cells exposed to the allergen (UT+A). The results are shown in **Fig. 6****,** with corresponding statistical analysis (Tukey HSD) in **Table 9.**

**Table 9. Statistical analysis of Der p1 study groups**

| | **UT** | **UT+A** | **#3** | **#4** | **#5** | **#15** | **#16** | **#25** | **#26** |
|---|---|---|---|---|---|---|---|---|---|
| **UT** | | p<0.01 | p<0.01 | p<0.01 | p<0.05 | p<0.01 | p<0.01 | NS | NS |
| **UT+A** | p<0.01 | | p<0.01 | p<0.01 | p<0.01 | p<0.01 | p<0.01 | p<0.01 | p<0.01 |
| **#3** | p<0.01 | p<0.01 | | NS | p<0.05 | NS | NS | p<0.01 | p<0.01 |
| **#4** | p<0.01 | p<0.01 | NS | | p<0.01 | NS | NS | p<0.01 | p<0.01 |
| **#5** | p<0.05 | p<0.01 | p<0.05 | p<0.01 | | NS | p<0.05 | p<0.01 | p<0.01 |
| **#15** | p<0.01 | p<0.01 | NS | NS | NS | | NS | p<0.01 | p<0.01 |
| **#16** | p<0.01 | p<0.01 | NS | NS | p<0.05 | NS | | p<0.01 | p<0.01 |
| **#25** | NS | p<0.01 | p<0.01 | p<0.01 | p<0.01 | p<0.01 | p<0.01 | | NS |
| **#26** | NS | p<0.01 | p<0.01 | p<0.01 | p<0.01 | p<0.01 | p<0.01 | NS | |

| | | | | | | | | | |
|---|---|---|---|---|---|---|---|---|---|
| NS non-significant | | | | | | | | | |

The results show that the compositions with combinations of partially crosslinked polymers (#25, #26) had a more significant mucoprotective anti-inflammatory effect against the Der p1 allergen compared to the compositions with non-crosslinked or single polymers (#4, #5, #15, #16, #27). The extent of these differences was surprising, suggesting that the alginate/carrageenan/CaCl₂ partially crosslinked combination may have a synergistic antiallergic mucoprotective effect.

The results further show dose-dependency and specificity of the tested effects, revealed in higher IL-8 expression under the higher concentration of the compositions compared to the lower concentration of the same compositions (x1 vs. x0.5).

The allergen challenge model was further used to test additional allergens (dust mite allergen Der f1 and pollen allergens Phl p1 and Car b1). Der f1 and Phl p1 allergens were tested in A549 cells treated with either the composition with the partially crosslinked polymers (#26) or the composition with the non-crosslinked polymers (#16), comparing treated and untreated cells (UT) and allergen exposed cells (UT+A). Car b1 allergen was tested in the same model, using various Car b1 concentrations (15 µg/ml, 30 µg/ml, 60 µg/ml). . The results are shown in **Figs 7A-7B** and **Fig. 8** (means ±SE in each group), with corresponding statistical analyses (Tukey HSD) in **Tables 10-12.**

**Table 10. Statistical analysis of Der f1 study groups**

| | **UT** | **UT+A** | **#16** | **#26** |
|---|---|---|---|---|
| **UT** | | p<0.01 | p<0.01 | p<0.01 |
| **UT+A** | p<0.01 | | p<0.01 | p<0.01 |
| **#16** | p<0.01 | p<0.01 | | p<0.01 |
| **#26** | p<0.01 | p<0.01 | p<0.01 | |

| | | | | |
|---|---|---|---|---|
| NS non-significant | | | | |

**Table 11. Statistical analysis of Phl p1 study groups**

| | **UT** | **UT+A** | **#16** | **#26** |
|---|---|---|---|---|
| **UT** | | p<0.01 | NS | NS |
| **UT+A** | p<0.01 | | p<0.01 | p<0.01 |
| **#16** | NS | p<0.01 | | NS |
| **#26** | NS | p<0.01 | NS | |

| | | | | |
|---|---|---|---|---|
| NS non-significant | | | | |

**Table 7. Statistical analysis of Car b1 study groups**

| | **UT** | **UT+A (µg/ml)** | **UT+A (µg/ml)** | **UT+A (µg/ml)** | **#16+A (µg/ml)** | **#16+A (µg/ml)** | **#16+A (µg/ml)** | **#26+A (µg/m)l** | **#26+A (µg/m)l** | **#26+A (µg/m)l** |
|---|---|---|---|---|---|---|---|---|---|---|
| | | **(60)** | **(30)** | **(15)** | **(60)** | **(30)** | **(15)** | **(60)** | **(30)** | **(15)** |
| **UT** | | p<0.01 | p<0.01 | p<0.01 | p<0.01 | p<0.01 | NS | p<0.01 | NS | p<0.01 |
| **UT+A 60 µg/ml** | p<0.01 | | NS | p<0.01 | p<0.01 | p<0.01 | p<0.01 | p<0.01 | p<0.01 | p<0.01 |
| **UT+A 30 µg/ml** | p<0.01 | NS | | p<0.01 | p<0.01 | p<0.01 | p<0.01 | p<0.01 | p<0.01 | p<0.01 |
| **UT+A 15 µg/ml** | p<0.01 | p<0.01 | p<0.01 | | p<0.01 | p<0.01 | p<0.01 | p<0.01 | p<0.01 | p<0.01 |
| **#16+A 60 µg/ml** | p<0.01 | p<0.01 | p<0.01 | p<0.01 | | p<0.01 | p<0.01 | NS | p<0.01 | p<0.01 |
| **#16+A 30 µg/ml** | p<0.01 | p<0.01 | p<0.01 | p<0.01 | p<0.01 | | p<0.01 | NS | p<0.01 | p<0.01 |
| **#16+A 15 µg/ml** | NS | p<0.01 | p<0.01 | p<0.01 | p<0.01 | p<0.01 | | p<0.01 | NS | p<0.01 |
| **#26+A 60 µg/ml** | p<0.01 | p<0.01 | p<0.01 | p<0.01 | NS | NS | p<0.01 | | p<0.01 | p<0.01 |
| **#26+A 30 µg/ml** | NS | p<0.01 | p<0.01 | p<0.01 | p<0.01 | p<0.01 | NS | p<0.01 | | p<0.01 |
| **#26+A 30 µg/ml** | p<0.01 | p<0.01 | p<0.01 | p<0.01 | p<0.01 | p<0.01 | p<0.01 | p<0.01 | p<0.01 | |

| | | | | | | | | | | |
|---|---|---|---|---|---|---|---|---|---|---|
| NS non-significant | | | | | | | | | | |

The results show that the anti-inflammatory mucoprotective effect of the compositions was not restricted to a specific allergen or a type of allergens, but were broadly applicable to different types of antigens, with the examples of several dust mite and pollen antigens. For all tested antigens, the composition with the partially crosslinked polymers was more effective than the composition with non-crosslinked polymers (#26 vs. #16).

Overall, present studies in both types of models, the viral infection and the allergen challenge, point to the surprising ability of the compositions with the combination of partially crosslinked polymers to induce robust and effective antiviral and anti-inflammatory mucoprotective effects.

### EXAMPLE 5: Transformability into a sprayable and durable film

Physical properties of the compositions and specifically their film forming ability at RT were also tested, measuring viscosity of the compositions with non-crosslinked (#16) and partially crosslinked (#26) polymers (diluted 1:1) over a prolonged period of time (21 days) by a viscometer (MRC, pedal No. 2 at 60 RPM, RT). The results are shown in **Fig. 9****.**

The results show that while both types of the compositions had a sustainable or constant viscosity at RT over the tested period, the partially crosslinked composition had a lower viscosity (by up to 20%), suggesting a better spray-ability, coverage and transformability into a spreadable, uniform and continuous film. This surprising physical property of a lower viscosity of the partially crosslinked compositions, and as a result their optimal spray-ability and coverage with no risk of clogging, makes these compositions particularly advantageous for topical and especially nasal applications.

The durability and dependability of the formed film was further tested measuring the viscosity under varying sheared forces at RT, including the compositions with single (#4, #5, #27) and the combinations of partially crosslinked (#25, #26) or non-crosslinked polymers (#15) tested under varying shear rate (0.1, 1, 10 s⁻¹) by a rheometer (NETZSCH Kinexus Pro, Malvern, discs 21, 61 mm, RT). The results are shown in **Fig. 10****.**

The results show that the viscosity of the compositions with combinations of the partially crosslinked polymers (#25, #26) was relatively resistant to shear forces (up to about 25% deviation) compared to the other compositions wherein the viscosity fluctuated dramatically under the same conditions. This study has provided additional proof of advantageous physical properties of the partially crosslinked compositions and their ability to form a dependable, durable and continuous film at RT.

Overall, these studies revealed several surprising and advantageous properties of the partially crosslinked compositions, manifested in a relatively constant or a sustained viscosity at RT over a prolonged period, which was also lower that the viscosity of the non-crosslinked and other compositions (by at least 20%) and was further resistant to varying shear forces (within the range of about 25%). This surprising and advantageous property of a superior viscosity is translated into the ability of these compositions to form (or transformability into) a sprayable, uniform, continuous and dependable film, which in turn, is responsible for the dramatic mucoprotective effects of these compositions.

### EXAMPLE 6: Development of inhalation and nasal products

Current efforts are focusing on the development of inhalation and nasal products. **Fig. 11** provides a schematic illustration of further embodiments of the presently disclosed compositions, including combinations of additional optional polymers, crosslinkers and preservatives. Additional embodiments include compositions in a form of a particulate matter, with particles or droplets in a specific size range that is optimal for nasal and inhalation applications (e.g., larger than 10 µm). This type compositions are currently being tested as per the specific requirements of FDA and EMEA for nasal and inhalation products.

### SPECIFIC EMBODIMENTS:

In the aspect of compositions according to the invention, it is meant that:
In some embodiments the compositions can be applied for delivery onto an epithelial mucosa.

In some embodiments the epithelial mucosa can be oral and/or nasal mucosa.

In some embodiments the compositions can comprise at least one sulfated polysaccharide polymer and at least one hydrophobic or hydrophilic polymer that are partially crosslinked by at least one positively charged ion, and wherein upon contact the mucosa, the composition is transformable into a continuous film.

In some embodiments the compositions can have an essentially sustained viscosity under varying shear forces.

In some embodiments the compositions can have a viscosity in a range between about 100-1000 mPa*sec⁻¹, measured under at a shear rate between about 0.1-10 s⁻¹ at room temperature.

In some embodiments the compositions can comprise micro- or nanoparticles.

In some embodiments the at least one sulfated polysaccharide polymer and the at least one hydrophobic or hydrophilic polymer can be synthetic, semisynthetic and/or modified natural polymers.

In some embodiments the at least one sulfated polysaccharide polymer and the at least one hydrophobic or hydrophilic polymer can be natural polymers obtained from a plant, an animal and/or a microbial origin.

In some embodiments the at least one sulfated polysaccharide polymer can be selected from galactans, ulvans, fucan, fucoidans, heparins, sulfated glucosamines, sulphated chitosan, chitin and/or chondroitin and sulfated dextran, or a combination thereof.

In some embodiments the at least one sulfated polysaccharide polymer can be at least one carrageenan or a combination thereof.

In some embodiments the at least one carrageenan can be selected from iota carrageenan, kappa carrageenan and/or lambda carrageenan.

In some embodiments the at least one hydrophobic or hydrophilic polymer can be selected from poly(glycolic acid) (PGA), poly(lactic acid) (PLA), polydioxanoes, polyoxalates, poly(α-esters), polyanhydrides, polyacetates, polycaprolactones, poly (orthoesters), polyamino acids, polyaminocarbonates, polyurethanes, polycarbonates, polyamides, poly(alkyl cyanoacrylates), mixtures and copolymers thereof.

In some embodiments the at least one hydrophobic or hydrophilic polymer can be selected from stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates, lactide/glycolide co-polymers, polyanhydrides and combinations thereof.

In some embodiments the at least one hydrophilic polymer can be a natural or synthetic hydrophilic polymer selected from polyethylene oxide (PEO), pullulan, hydroxypropylmethyl cellulose (HPMC), hydroxyethyl cellulose (HPC), hydroxypropyl cellulose, polyvinyl pyrrolidone, carboxymethyl cellulose, polyvinyl alcohol, sodium alginate, polyethylene glycol, xanthan gum, tragancanth gum, guar gum, acacia gum, arabic gum, polyacrylic acid, methylmethacrylate copolymer, carboxyvinyl copolymers, starch, gelatin, ethyl cellulose, hydroxypropyl ethyl cellulose, cellulose acetate phthalate, hydroxypropyl methyl cellulose phthalate and combinations thereof.

In some embodiments the at least one hydrophilic polymer can be a natural polymer which is a polysaccharide or a protein.

In some embodiments the polysaccharide can be selected from selected from dextrans, alginates, chitosans, agaroses and/or pullulans.

In some embodiments the protein can be selected from albumins, gelatins, collagens, lectins, legumines, and/or vicilines.

In some embodiments the polysaccharide can be alginate or an alginate salt.

In some embodiments the at least one positively charged ion can be a di-valent cation.

In some embodiments the di-valent cation can be selected from Ba²⁺, Be²⁺, Ca²⁺, Co²⁺, Mg²⁺, Cu²⁺, Ni²⁺, Fe²⁺ and/or Zn²⁺.

In some embodiments the at least one sulfated polysaccharide polymer can be a carrageenan and the at least one hydrophilic polymer can be an alginate that are partially crosslinked by Ca²⁺.

In some embodiments the carrageenan can be at a concentration in the range between about 0.005% and about 1%, the alginate can be at a concentration in the range between about 0.1% and about 3%, and the Ca²⁺ can be at a concentration in the range between about 0.0001% and about 1% (w/w).

In some embodiments the carrageenan can be at a concentration in the range between about 0.1% and about 0.3%, the alginate can be at a concentration in the range between about 1% and about 3%, and the Ca²⁺ can be at a concentration in the range between about 0.0001% and about 0.05% (w/w).

In some embodiments the carrageenan can be at a concentration in the range between about 0.2% and about 0.3%, the alginate can be at a concentration in the range between about 2.0% and about 2.9%, and the Ca²⁺ can be at a concentration in the range between about 0.0001% and about 0.02% (w/w).

In some embodiments the composition can further comprise a solvent, a buffer, an excipient, an adjuvant, a permeation enhancer, a plasticizer, a thickener, a rheological modifier, an alcohol, an extender, a preservative, a colorant, a pigment, a sweetener, a flavoring and/or an odor agent, or combinations thereof.

In some embodiments the compositions can further comprise at least one additional therapeutic agent.

In some embodiments the compositions can be pharmaceutical compositions comprising a therapeutically effective amount of any one of the compositions as above and a pharmaceutically acceptable buffer, carrier or an excipient.

In some embodiments the pharmaceutical compositions can be adapted for inhalation, optionally in the form of particles or droplets.

In some embodiments the pharmaceutical compositions can be adapted for oral and/or nasal administering, optionally in the form of a spray and/or a gel.

In some embodiments the pharmaceutical compositions can further comprise at least one additional therapeutic agent.

In some embodiments the compositions can further comprise at least one therapeutic agent for preventing or treating a disorder related to oral and/or nasal mucosal barrier dysfunction.

In some embodiments the compositions can be used for protecting oral and nasal mucosal barrier function.

In some embodiments the compositions can be used for treating, alleviating and/or preventing a microbial oral and/or nasal infection.

In some embodiments the microbial oral/and/or nasal infection can be a viral and/or a bacterial infection.

In some embodiments the compositions can further comprise at least one antibiotic and/or antiviral drug.

In some embodiments the antiviral drug can be an antiviral vaccine.

In some embodiments the compositions can be used for treating, alleviating and/or preventing an aeroallergen induced allergy and/or inflammation.

In some embodiments the composition can comprise at least one anti-allergic and/or anti-inflammatory drug.

In some embodiments the compositions can be used for regeneration of a tissue and/or in bioengineering an implant for a tissue, it is meant that:
In some embodiments the tissue can be a neuronal or a cardiac tissue.

In the aspect of kits, it is meant that:
In some embodiments the kits can comprise any one of the compositions or pharmaceutical compositions as above and a device for oral or nasal delivery or inhalation, and instructions for use.

In the aspect of drug delivery systems, it is meant that:
In some embodiments the drug delivery systems can comprise any one of the compositions as above and at least one additional therapeutic agent.

In some embodiments the at least one therapeutic agent can be an agent for preventing or treating a disorder or a condition related to oral and/or nasal mucosal barrier dysfunction.

In some embodiments the at least one therapeutic agent can be an agent for preventing or treating other types of clinical disorders or clinical and subclinical conditions.

In some embodiments the at least one therapeutic agent can be selected from antibiotics, antifungal agents, antiviral agents, neuroleptics, analgesics, hormones, anti-inflammatory drugs, non-steroidal anti-inflammatory drugs, anti-rheumatic, drugs anticoagulants, beta-blockers, diuretics, anti-hypertension drugs, anti-atherosclerosis drugs, antidiabetics, anti-asthmatic drugs, decongestant and/or cold medicines.

In the aspect of methods for oral or nasal delivery, it is meant that:
In some embodiments the methods can comprise an oral or a nasal administering of a therapeutically effective amount of any one of the compositions, or drug delivery systems as above.

In some embodiments the methods can further comprise co-administering of at least one additional therapeutic agent.

In some embodiments the methods can be applied for protecting oral and nasal mucosal barrier function.

In some embodiments to the methods can be applied for treating, alleviating, and preventing a microbial oral and/or nasal infection.

In some embodiments the methods can further comprise concomitant administering of at least one antibiotic and/or antiviral drug, said administering being via oral, enteral, parenteral, or nasal routes.

In some embodiments the methods can be applied for preventing, alleviating or treating an aeroallergen induced allergy and/or inflammation.

In some embodiments the methods can comprise concomitant administering of at least one anti-allergic and/or anti-inflammatory drug, said administering being via oral, enteral, parenteral, or nasal routes.

In some embodiments the methods can be applied for regeneration of a tissue and/or in bioengineering an implant for a tissue.

In some embodiments the methods can further comprise a surgical procedure.

In the aspect of use, it is meant that:
In some embodiments the compositions as above can be used for the manufacture of a medicament for preventing, alleviating or treating an aeroallergen induced allergy or inflammation.

In some embodiments the medicament can be for preventing, alleviating or treating a microbial oral and/or nasal infection.

In some embodiments the medicament can be for treating a systemic disorder.

### ANNEX A

### List of general drug categories according to the FDA

**Analgesics** including non-narcotic and narcotic analgesics
**Antacids**
**Antianxiety Drugs**
**Antiarrhythmics**
**Antibacterial agents**
**Antibiotics** including naturally occurring, synthetic, broad-spectrum antibiotics
**Anticoagulants and Thrombolytics** for arterial or venous thrombosis
**Anticonvulsants**
**Antidepressants** including mood-lifting antidepressants: tricyclics, monoamine oxidase inhibitors, and SSRIs
**Antidiarrheals** including antidiarrheal preparations and drugs that slow down the contractions of the bowel muscles
**Antiemetics**
**Antifungals** including infections that affect hair, skin, nails, mucous membranes
**Antihistamines**
**Antihypertensives** including diuretics, beta-blockers, calcium channel blocker, ACE (angiotensin- converting enzyme) inhibitors
**Anti-Inflammatories**
**Antineoplastics**
**Antipsychotics** also major tranquilizers
**Antipyretics**
**Antivirals** including treatment and temporary protection against viral infections
**Barbiturates** (see sleeping drugs).
**Beta-Blockers**
**Bronchodilators**
**Cold Cures** in relation to aches, pains, and fever that accompany a cold
**Corticosteroids** in the context of immunosuppression, malignancies, or deficiency disorders
**Cough Suppressants** including narcotic and non-narcotic suppressants
**Cytotoxics** as antineoplastics and as immunosuppressives
**Decongestants**
**Diuretics**
**Expectorant**
**Hormones:** including synthetic equivalents and natural hormone extracts
**Hypoglycemics (Oral)**
**Immunosuppressives**
**Laxatives**
**Muscle Relaxants** including those that relieve muscle spasm and minor tranquilizers
**Sedatives**
**Sex Hormones (Female)** including those used for menstrual and menopausal disorders, oral contraceptives, and for treating female and male cancers.
**Sex Hormones (Male)** including those used for male hormonal deficiency in hypopituitarism or disorders of the testes, also for treating cancer, and anabolic steroids
**Sleeping Drugs**
**Tranquilizer** including minor and major tranquilizers
**Vitamins**

### ANNEX B

### List of spectra of diseases/conditions according to the FDA

### Addiction

- Maintenance of abstinence from alcohol and/or drugs in patients with alcohol or drug dependency
- Neonatal abstinence syndrome (NAS)
- Smoking cessation

### Allergy

- Type I/II hereditary angioedema

### Analgesia/Anesthesiology/Anti-inflammatory

- Anesthesia
- Anxiolysis
- Fever reduction
- Pain
   ∘ Chronic
   ∘ Malignancy-related breakthrough pain
   ∘ Mild
   ∘ Moderate
   ∘ Post-operative
   ∘ Severe
- Reversal of neuromuscular blockage
- Sedation
   ∘ Initially intubated & mechanically ventilated pediatric subjects in an intensive care setting
- Skeletal muscle relaxation to facilitate rapid sequence and routine endotracheal intubation

### Cardiovascular Disease

- Acute coronary disease
- Congenital heart disease
- Congestive heart failure
   ∘ heart failure due to systemic left ventricular systolic dysfunction
- Dilated Cardiomyopathy (DCM) and symptomatic chronic heart failure
- Hypertension
   ∘ General
   ∘ Post-operative hypertension
- Pulmonary hypertension
   ∘ Primary/secondary
   ∘ Post-surgical
- Raynaud's Phenomenon
- Tachyarrhythmia

### Dermatology

- Alopecia areata
- Aphthous ulcer
- Candidiasis (cutaneous, oropharyngeal)
- Dermatosis (steroid-responsive dermatosis)
- Dermatitis (atopic dermatitis)
- Ichthyosis vulgaris
- Idiopathic urticaria (chronic)
- Mild to moderate plaque psoriasis
- Molluscum contagiosum
- Onychomycosis of the toenail
- Oral mucositis
- Pediculosis humanis capitis (head lice and their ova)
- Severe recalcitrant nodular acne
- Skin and skin structure infections
- Tinea
   ∘ Capitis
   ∘ Cruris
   ∘ Pedis

### Endocrinology

- Acromegalic gigantism
- Bone mineral density (BMD)
   ∘ BMD in anorexia nervosa patients
   ∘ BMD in osteogenesis imperfecta patients
   ∘ BMD in postmenarcheal adolescents with secondary amenorrhea
- Central Precocious Puberty (CPP)
- Delayed puberty in boys with primary and secondary hypogonadism
- Diabetes Mellitus
   ∘ Type 1
   ∘ Type 2
- Failure to thrive in pediatric AIDS patients
- Growth-hormone-secreting lesions of the pituitary gland
- Gynecomastia
- Homozygous familial hypercholesterolemia (HoFH)
- Hypercholesterolemia (heterozygous familial hypercholesterolemia)
- Hyperparathyroidism (renal failure secondary to hyperparathyroidism)
- Hypocalcemia management in patients on hemodialysis
- McCune-Albright Syndrome
- Obesity
   ∘ General
   ∘ Hypothalamic
- Osteogenesis Imperfecta (OI)
- Tetrahydrobiopterin- (BH4-) responsive phenylketonuria (PKU)
- Testotoxicosis

### Gastroenterology

- Antiemesis
   ∘ Treatment and prevention of nausea and vomiting associated with emetogenic chemotherapy
   ∘ Prevention of post-operative nausea and vomiting
- Clostridium difficile-associated diarrhea (CDAD)
- Constipation (general)
- Crohn's Disease
- Diarrhea (acute)
- Erosive Esophagitis
- Gastroesophageal Reflux Disease (GERD)
- Irritable Bowel Syndrome (IBS)
   ∘ IBS-D
- Nephropathic cystinosis
- Short Bowel Syndrome (SBS)
- Ulcerative Colitis (UC)
- Ulcers

### Hematology/Coagulation

- Deep vein thrombosis (DVT)
- Iron deficiency in patients undergoing chronic hemodialysis
- Iron overload due to blood transfusions-dependent anemia (chronic)
- Sickle cell disease
- Thrombocythemia
- Thrombocytopenia
- Thromboembolism

### Immunomodulators

- Immune suppression
- Prevention of organ rejection following renal transplantation

### Infectious Disease (viral)

- Cytomegalovirus (CMV)
- Hepatitis B virus (HBV)
   ∘ Chronic
- Hepatitis C virus (HCV)
   ∘ Chronic
- Herpes simplex virus (HSV)
- Human Immunodeficiency Virus (HIV) infection
   ∘ prophylaxis of HIV infection in exposed neonates
   ∘ perinatal transmission of HIV infection
- Influenza A/B
- Perinatal transmission of HIV infection
- Symptoms associated with common cold and influenza
- Varicella zoster virus (VZV)
- COVID-19 (SARS-CoV-2)

### Infectious Disease (non-viral)

- Acute pyelonephritis
- Aspergillosis (invasive)
- Candidiasis
- Cerebrospinal fluid (CSF) shunt infection
- Community-acquired pneumonia (CAP)
- Complicated intra-abdominal infections (cIAI)
- Complicated UTI
   ∘ Acute pyelonephritis
- Fungal infection (invasive)
   ∘ Associated fever and neutropenia
- *Helicobacter pylori* infection *(H. pylori)*
- Malaria
- Meningitis (bacterial)
- Mold infections (rare mold)
- Otitis media (OM)(recurrent)/OM treatment failures
- Pneumonia (bacteremic)
- Resistant infection or infections unresponsive to the first-choice antibiotic
- Skin and skin structure infections
- Tuberculosis

### Neurology

- Congenital and non-congenital neurological conditions
- Epilepsy
- Duchene muscular dystrophy
- Insomnia in patients with ADHD
- Intractable seizures associated with Dravet syndrome
- Migraine
- Multiple sclerosis (MS)
   ∘ Relapsing forms of MS
- Narcolepsy
   ∘ Cataplexy in narcolepsy
- Neuroblastoma
   ∘ High-risk refractory or relapsed neuroblastoma
- Neuromuscular disorders
- Seizures
   ∘ Generalized
   ∘ Lennox-Gastaut Sundrome (LGS)
   ∘ Partial
      ▪ with or without secondarily generalized seizures
- Spasticity management
- Obstructive sleep apnea (OSA) (excessive sleepiness in OSA)
- Tourette's syndrome
   ∘ ADHD in patients with Tourette's disorder

### Oncology

- Allogeneic bone marrow transplants
- Chemotherapy toxicity protection
- Chronic Graft Versus Host Disease (GVHD)
- CNS malignancies and solid tumors
- Giant cell tumor of bone and osteosarcoma
- Hematologic tumors
   ∘ CNS lymphomas
      ▪ Low grade gliomas
      ▪ High grade gliomas
      ▪ Recurrent, progressive, or refractory brain tumors
   ∘ Leukemia
      ▪ Acute lymphoblastic leukemia
      ▪ Acute myelogenous leukemia
      ▪ CNS leukemia
      ▪ Mixed linear acute leukemia
      ▪ Philadelphia positive (Ph+) chronic myelogenous leukemia
   ∘ Hodgkin's lymphoma
   ∘ Non-Hodgkin's lymphoma
      ▪ Anaplastic large cell lymphoma
      ▪ Mature B-cell NHL
- Hematopoietic stem cell mobilization in patients with pediatric malignancies eligible for autologous stem cell transplantation
- Melanoma
- Myelodysplastic syndromes
- Neuroblastoma
- Osteosarcoma
- Pediatric malignancies
- Refractory/relapse malignancies
   ∘ advanced relapsed/refractory malignant solid tumors with activation of the RAS/RAF/MEK signaling pathway.
   ∘ relapsed or refractory solid tumors containing BRAF V600 activating mutations and in the treatment of adolescent patients with unresectable or metastatic melanoma containing BRAF V600 activating mutations.
   ∘ relapsed or refractory tumors with Erb-B1 or Erb-B2 pathway dysregulation.
   ∘ Rhabdomyosarcoma and non-rhabdomyosarcomatous soft tissue sarcoma.

### Ophthalmology

- Conjunctivitis
   ∘ Allergic
   ∘ Bacterial
   ∘ Neonatal
- Intraocular pressure
- Post-operative inflammation following cataract surgery
- Retinopathy of prematurity (ROP)
- Uveitis

### Psychiatry

- Adolescent schizophrenia
- Attention deficit hyperactivity disorder (ADHD)
- Autism and Autism Spectrum Disorder
   ∘ Core social impairment symptoms
- Bipolar disorder (mania associated with bipolar disorder)
- Depression/Major Depressive Disorder (MDD)
- General Anxiety Disorder (GAD)
- Obsessive compulsive disorder (OCD)
- Panic disorder
- Alzheimer's and Parkinson's disease

### Pulmonary

- Allergic Rhinitis
- Asthma
- Bronchopulmonary dysplasia
- Bronchospasm (treatment and prevention)
   ∘ Prevention of bronchospasm in patients with obstructive airway disease
- Cystic Fibrosis

### Renal Disease

- Anemia due to chronic kidney disease (CKD)
- End stage renal disease
- Hyperphosphatemia due to chronic kidney disease
- Hyponatremia

### Rheumatology

- Familial Mediterranean Fever (FMF)
- Juvenile Idiopathic Arthritis (JIA)/Juvenile Rheumatoid Arthritis (JRA)
   ∘ Polyarticular Juvenile Idiopathic Arthritis (PJIA)
- Management of fibromyalgia

### Urologic

- Detrusor Hyperreflexia
- Detrusor overactivity in neurological condition

## Claims

1. A drug delivery system comprising a composition for delivery onto an epithelial mucosa and at least one therapeutic agent,
wherein the composition comprises at least one sulfated polysaccharide polymer and at least one hydrophobic or hydrophilic polymer that are partially crosslinked by at least one positively charged ion, and
wherein the composition has a substantially constant viscosity at room temperature (RT), whereby upon contact with the epithelial mucosa the composition is transformable into a uniform and continuous film.

2. The drug delivery system of claim 1, wherein the epithelial mucosa is oral and/or nasal mucosa.

3. The drug delivery system of claim 1, wherein before contact with the epithelial mucosa, the composition is in a liquid, a semiliquid or sprayable form.

4. The drug delivery system of claim 1, wherein the viscosity remains substantially constant within a range between about 1 mPa*s⁻¹ and about 20 mPa*s⁻¹ for a period of at least 10 days or more, as measured by a viscometer.

5. The drug delivery system of claim 1, wherein the viscosity remains substantially constant when measured under varying shear forces and has less than 25% deviation for varying shear rates in the range between about 0.1 and about 10 s⁻¹.

6. The drug delivery system of claim 1, comprising micro or nano particles or droplets.

7. The drug delivery system of claim **1,** wherein the at least one sulfated polysaccharide polymer is selected from sulfated galactans, ulvans, fucan, fucoidans, heparins, glucosamines, dextrans, chitosan, chitin and chondroitin.

8. The drug delivery system of claim **1,** wherein the at least one sulfated polysaccharide polymer is at least one carrageenan selected from iota carrageenan, kappa carrageenan and lambda carrageenan.

9. The drug delivery system of claim **1,** wherein the at least one hydrophilic polymer is a polysaccharide selected from dextrans, alginates, chitosans, agaroses and pullulans, or a protein selected from albumins, gelatins, collagens, lectins, legumines, and vicilines.

10. The drug delivery system of claim **1,** wherein the at least one hydrophilic polymer is alginate or an alginate salt.

11. The drug delivery system of claim **1,** wherein the at least one positively charged ion is a di-valent cation selected from Ba²⁺, Be²⁺, Ca²⁺, Co²⁺, Mg²⁺, Cu²⁺, Ni²⁺, Fe²⁺ and Zn²⁺.

12. The drug delivery system of claim **1,** wherein the at least one sulfated polysaccharide polymer is a carrageenan and the at least one hydrophilic polymer is an alginate and the at least one positively charged ion is Ca²⁺.

13. The drug delivery system of claim **12,** wherein the carrageenan is at a concentration in a range between about 0.005% and about 1%, the alginate is at a concentration in a range between about 0.1% and about 3%, and the Ca²⁺ is at a concentration in a range between about 0.0001% and about 1% (w/w).

14. The drug delivery system of claim **1,** wherein the at least one therapeutic agent is an agent for preventing or treating a clinical disorder or a clinical or subclinical condition.

15. The drug delivery system of claim **1,** wherein the at least one therapeutic agent is selected from antibiotics, antifungal agents, antiviral agents, neuroleptics, analgesics, hormones, anti-inflammatory drugs, non-steroidal anti-inflammatory drugs, anti-rheumatic, drugs anticoagulants, beta-blockers, diuretics, anti-hypertension drugs, anti-atherosclerosis drugs, antidiabetics, anti-asthmatic drugs, decongestant and/or cold medicines.
